# EUROPEAN PATENT SPECIFICATION

(11) **EP 4 512 585 B1**
(45) Date of publication and mention of the grant of the patent: **11.11.2026**
(21) Application number: 23193370.6
(22) Date of filing: 25.08.2023
(51) Int. Cl.: B26D 3/16, B26D 7/27

(54) **CUTTING DEVICE FOR TUBE DISCONNECTION AND METHOD OF PREPARING TUBE DISCONNECTION**
SCHNEIDVORRICHTUNG ZUR ROHRTRENNUNG UND VERFAHREN ZUR HERSTELLUNG EINER ROHRTRENNUNG
DISPOSITIF DE COUPE POUR DÉCONNEXION DE TUBE ET PROCÉDÉ DE PRÉPARATION DE DÉCONNEXION DE TUBE

(43) Date of publication of application: 26.02.2025
(73) Proprietor: Sartorius Stedim FMT, 13400 Aubagne (FR)
(72) Inventor: Unterlechner, Christian, 6122 Fritzens (AT)
(74) Representative: Novagraaf International SA

(56) References cited:
- WO-A1-2014/032764
- WO-A1-2022/133105
- FR-A1- 2 939 348

## Description

### FIELD OF THE INVENTION

The present disclosure relates generally to systems having tubes, and more particularly, a tube disconnection system and a method for crimping a tube section of a tube and allowing a cut for separating two tube members.

### BACKGROUND OF THE INVENTION

In the pharmaceutical, chemical, and other files that use tubing in the processing and transport for fluids (such as biopharmaceutical fluids), there is often a need for separating two tube members of the tubing, by applying a sealing action and cutting. This enables to seal off and isolate the fluids within from contamination from the external environment. In biopharmaceutical production, drugs are produced and stored in single use bags. After production, bags or transfer lines also need to be disconnected to allow a transfer process.

A known method requires a cylindrical metal collar or sleeve, which is placed over a portion of the tube, before using a tool to deform and cut the collar and the tube, thus forming a crimped end. Document WO 2022/133105 discloses a cutting operation performed after mounting a slotted sleeve that is made of malleable and rigid material, so that the sleeve can be placed over large bioprocess tubes. The pair of adjacent separating edges, defining the elongated slot in the sleeve, allow the sleeve to be slidably mounted onto a bioprocess tube. Once positioned on the bioprocessed tube, a cutting operation takes place that goes through the separating collar and the bioprocess tube. This is resulting in a sterile disconnect.

Drawbacks of such sleeve mounting is that the sleeve needs to be placed early in the process (typically in the manifold production) and it is not possible to cut metal sleeve in big diameter. Additionally, it has been observed the metal portions will have a sharp edge after the cutting, causing a risk of injury.

Other tube disconnection systems exist, with a great variety in the way the tubing is handled. Some methods use a guillotine-like cutting tool to separate two hose sections, which is guided approximately perpendicularly to the longitudinal direction of the hose during the cutting process. Document WO 2014/032764 A1 discloses a method with such tool for cutting a tube encasement, with the cutting area circumferentially covered by a strip of material having an adhesive on strip inner side, before using the cutting tool. Some methods use thermo-sealing, which generally increase operation duration and requires a higher cost equipment.

There is still a room for improving operations that relate to such tubes, especially by cutting them into sections that are closed permanently, while keeping a tube end aseptic, i.e., with their bacterial integrity not challenged.

### OBJECTS AND SUMMARY OF THE INVENTION

For improving situation, the invention, which is defined in claim 1, provides a cutting device for cutting a flexible tube at a tube section thereof, the cutting device comprising:
- at least one cutting member;
- a band assembly comprising a first crimping member adjacent to a band assembly cutting site;
- a first tube holder;
- a second tube holder, the first tube holder and the second tube holder being a pair of tube holders adapted to delimit a housing for receiving the tube section of the flexible tube to be cut;
- an interspace, located between the first tube holder and the second tube holder, the interspace separating a first holding region associated with the first tube holder from a second holding region associated with the second tube holder; and
- a driving assembly for driving the pair of tube holders from an open conformation, allowing installation of the tube section in the housing, to a closed conformation, in which the first tube holder is crimping the first crimping member and participates in pinching the tube section together with the second tube holder;
wherein the band assembly is adapted to delimit, before any crimping, a receiving part at least partly delimited by a surface (surface delimiting a recess, typically a generally concave surface, for instance with U-shaped profile) of the first crimping member for accommodation of the tube section;
and wherein the cutting device comprises positioning means, possibly arranged in the pair of tube holders, that are configured to maintain the band assembly inside the housing, in an interposed configuration for:
- covering the tube section extending in the receiving part on the one hand,
- and contacting at least one amongst the first tube holder and the second tube holder on the other hand, with the band assembly cutting site positioned at an intermediary position between the first tube holder and the second tube holder,
whereby a cutting operation performed for said interposed configuration, by the cutting member through the interspace, causes the tube section to be cut transversally at/in coincidence with the band assembly cutting site without cutting the first crimping member that is crimped in the closed conformation.

The tube section can thus be cut via a transverse cut (with the cutting member being guided in the device, optionally inside a casing of the device) that is also cutting the band assembly at the cutting site. The interspace may form a passageway allowing insertion of the cutting member to cut the flexible tube, due to a relative movement between the cutting member and the housing delimited by the tube holders. It is understood the tube section can be deformed radially when maintained in the housing, in the closed conformation, while being kept stationary at least along an axial direction parallel to the tube section.

With such device, the cutting site of the band assembly may extend selectively in the interspace (only at such interspace) and the band assembly may occupy a predetermined position obtained by a coupling action involving the positioning means. The band assembly also covers (partly covers) the tube section, for instance from below and/or so as to cover (at the crimping member) at least half the outer circumference of the tube section near/adjacent to the region to be cut. This ensures the crimping member is shifted relative to the real cutting site. The positioning means can be configured to interfere with placement of the band assembly in the housing, for instance with a guiding effect for a lower face of the band assembly. A mechanical coupling may occur to provide a stabilizing effect or fastening effect for the band assembly.

Thanks to the cutting device, a good trade-off is obtained between simplicity of the mechanism and ease of use (fast disconnection may be obtained), the driving assembly being actuatable to simultaneously perform the crimping and obtain the cut at the tube section, while also advantageously preventing cutting the crimping member(s) involved for the sealing at the tube cut end. A relatively low cutting force may be required.

Such solution is compatible with a great flexibility regarding:
- the area of the flexible tube to be cut, since there is no need for mounting in advance a sleeve,
- the outer diameter or similar characteristic size of the flexible tube, as the tube holders have an open conformation.
The cutting device may also be efficient without any increase of temperature, which means silicon tubes may be also cut with such solution.

For allowing the cutting, it is understood that, in the closed conformation, the first tube holder and the second tube holder are both pinching the tube section to obtain a sealed section of the tube on either side of the interspace. Such pinching includes a pinching exerted on the first crimping member, so that cutting may be performed as soon as or after a sealed state in obtained for the tube portion surrounded by the first crimping member.

The driving assembly may have a part inserted inside a casing that accommodates the tube holders (in an interior volume of the casing) at least in the closed conformation, while an outer end part of the driving assembly is available outside the casing.
In some variants, the driving assembly may essentially or totally extend outside the casing. Possibly, the driving assembly has one or two outer driving arms extending outside such casing and connected to a hinge mechanism or a mechanical linking portion linking jaws of the respective tube holders. The driving assembly may be actuated in an inverted direction, causing reciprocal motion to return to the open conformation of the tube holders, after obtaining the cut. Due to higher rigidity of the first crimping member as compared to the flexible tube plastic material, the crimped state is a permanently deformed state of the tube portion that includes a cut end, so that a closed tube end is obtained according to a sterile disconnection.

In some options, the cutting member may be heated or irradiated (by appropriate UV radiation or the like) to ensure sterility of the cutting edge or similar cutting part of the cutting member that is in contact with the flexible tube.

Independently from the specific way the tube holders are specifically driven, the interspace may form a passageway allowing insertion of the cutting member to cut the flexible tube, due to a relative movement between the cutting member and the housing delimited by the tube holders.

According to embodiments, the band assembly comprises two distinct crimping members, including the first crimping member and a second crimping member. In the closed conformation, the first tube holder and the second tube holder are crimping the two crimping members and pinching the tube section.

When having two crimping members, the receiving part for accommodation of the tube section may be delimited by two interior surfaces (each delimiting a recess) of the two crimping members, the two interior surfaces including the first surface (that may be a generally concave/inwardly hollowed surface). The positioning means may be configured to maintain inside the housing the band assembly in contact with the first tube holder and with the second tube holder, while the band assembly extends inside the receiving part.

The positioning means are functionally cooperating with at least one positioning part of the band assembly, preferably with the two crimping members, to have the two crimping members distributed respectively, preferably selectively distributed, in the first holding region and in the second holding region, whereby the cutting at the band assembly cutting site is a cutting performed in an intermediate region between the two crimping members for also cutting the tube section transversally without cutting any one of the two crimping members that are crimped in the closed conformation of the pair of tube holders.

In some options, the positioning means comprises one or two magnetic members. For instance, the magnetic members are adapted to magnetically interact with the two crimping members each constituted of or including a magnetizable material. Such arrangement may be suitable to have a flat portion of the crimping members magnetically attracted in predetermined regions of the housing, along dedicated surfaces of the tube holders.

In embodiments of the device, one or more of the following particulars may be provided:
- the driving assembly includes a separating part, optionally an end part at the opposite from an actuatable part of the driving assembly, interposed between the first tube holder and the second holder.
- a thickness of the separating part provided in the driving assembly, between the two tube holders, may be substantially equal to a distance provided between the two crimping members of the band assembly.

- the crimping members are each made of a malleable and rigid composition, optionally containing metal or other ferrous material.
- the crimping members have a substantially constant thickness, possibly except at relief(s) involved for a cooperation with the positioning means provided on contact surface(s) of the tube holders for direct contact with the band assembly.
- the cutting device forms a crimping tool, with an ease of installing the band assembly and the tube section of the flexible tube, with a (wide) open configuration of a pair of tube holders.
- the tube holders provide each a contact surface without teeth for contact with a bottom of a corresponding one of the crimping members.
- the first crimping member or each crimping member is U-shaped or C-shaped in a nominal configuration, before crimping by at least one of the tube holders.
- one of the positioning means include one or two reliefs included in a bottom part of a tube holder, allowing a mechanical fastening or a coupling using shape complementarity.
- two positioning means are provided and only one of the positioning means has a magnetic member, possibly providing a guiding effect.

In some options, each of the crimping members may be provided with protrusions or reliefs that cooperate mechanically, typically without any adhesive interaction, with the tube holders. This facilitates positioning of the band assembly (taken as a whole) in the housing, with understanding that gravity center of the band assembly may be typically at or adjacent to the cutting site.

In some options, at least one or several of the magnetic members included in the positioning means may be chosen amongst a permanent magnet, a plastic composition including magnetic powder or particles distributed in at least one layer of the magnetic member.
One of the tube holders or each tube holder may be provided with a lower jaw having a cavity or recess for accommodation of the magnetic member, so that the magnetic member is not protruding inside the receiving part.

According to a preferred embodiment for option using two crimping members in the band assembly, a minimal spacing distance between the two crimping members, preferably matching with the interspace just before a crimping due to displacement toward the closed conformation, is comprised between 1,5 and 10 mm.
The band assembly may be a 3-D device with each of the crimping members adapted to be crimped from an initial U-shaped conformation to an annular conformation that is flattened to maintain a sealed state at each corresponding cut end of the flexible tube.

After the cut, the tube end part/edge protruding from the crimping member at the cut end may have an axial extension inferior to 5 mm (measured along elongation direction of the tube portion). In some options, the spacing distance may be comprised between 2 and 6 mm. For instance, the tube end part/edge protruding from the crimping member at the cut end may have an extension (axial extension) inferior or equal to 2 or 3 mm.

In embodiments of the device, one or more of the following particulars may be provided:
- the crimping members, provided with a metal composition, are more rigid than a strip or band of the band assembly.
- the positioning means comprises a magnetic member adapted to magnetically interact with at least one amongst the two crimping members.
- the pair of tube holders is arranged substantially in alignment and spaced along a first direction in an operation mode of the cutting device, for the interposed configuration, so that the cutting operation performed by the cutting member is a cutting of the tube section perpendicular to the first direction.
- such cutting is performed through the interspace and/or at the band assembly cutting site.

According to a particular aspect, which is not claimed, taken alone or in line with what is mentioned above. there is provided a band assembly for use with a cutting device provided with a pair of tube holders and an interspace, the band assembly comprising one or two crimping members, which are preferably U-shaped and suitable to form a single receiving part for a tube section of a flexible tube to be cut. The band assembly also comprises an elongated flexible strip or band whose length is superior to a length of the housing delimited by the tube holders or superior to extension of the crimping members, knowing that the flexible strip or band is crossing/overlapping each bottom of the crimping member. The flexible strip or band is secured to each crimping member, on a same side thereof when two crimping members are provided.

More generally, such band assembly may constitute a separable part of the cutting device and can be produced independently/separately.

A band assembly is of interest to provide an efficient disconnection at a tube section, independently from the specific kind of cutting device involved. Embodiments of the disclosure provide a band assembly, which is not claimed, suitable in participating in a flexible tube disconnection by extending around a tube section (of annular shape) of the flexible tube to be disconnected by a cut, the band assembly comprising:
- a first crimping member, possibly under the form of a folded piece of a strip material,
- a second crimping member, possibly under the form of a folded piece of a strip material,
- a supporting part of flexible material secured to the first crimping member and secured to the second crimping member, the supporting part being preferably under form of a tape or band that is elongated along a first direction, the flexible material being less rigid than strip material of any one amongst the first crimping member and the second crimping member, wherein the band assembly is provided with a receiving part for accommodation of the tube section, the receiving part being delimited by two interior surfaces of the two crimping members, and wherein the supporting part is adapted to extend planar at least at a spacing region made of the flexible material and allowing, in a planar state of the spacing region, to maintain the first crimping member and the second crimping member mutually spaced, the first crimping member and the second crimping member having each a cross section opened on a same side that is at the opposite from the spacing region that includes one or more bridge portions extending between said crimping members.

Each crimping member may define a generally concave interior surface, either with a continuously curved surface (such as regularly curved C-shaped profile) or with angles (U-shaped profile). Two branches may be present in each crimping member, to obtain a generally concave profile in a cross-section view (which may correspond, just before the crimping operation, to a view along a plane perpendicular to the common axis of the tube holders that receive the band assembly).

In embodiments of the band assembly, any of the above identified structural features of the band assembly is used, independently from the way the cutting is performed and independently from the device holding parts (external with respect to the band assembly) involved in the crimping operation.

At least one of the crimping members is magnetizable and/or made of metal, with a thickness lower than 3 or 4 mm.

The spacing region belonging to the supporting part may have a maximal thickness inferior or equal to 2 mm. Possibly a maximal thickness of the supporting part is inferior to 2 mm, preferably inferior or equal to 1 mm. It is understood the supporting part material allows a bending, such material being flexible material or semi-rigid material (mainly flexible around a transverse axis for instance and almost not foldable for other axes).

The band assembly may be provided with one or two identifying devices, at least one identifying device allowing identification of the tube portion that is cut and surrounded by a cut portion of the band assembly. A label with a serial number may form all or part of the identifying device.

Optionally, the band assembly includes, in the supporting part, at an end of the supporting part or in an overlapped conformation with respect to the supporting part, a tape provided with an identifying device.
A tape of the band assembly, possibly belonging to the supporting part, may include or support two identifying devices. In some embodiments, the two identifying devices are label parts, optionally integrated as additional layer on the flexible band or included in a band layer that forms/includes the junction portion arranged adjacent the first crimping member and defining the cutting site and/or arranged between the two crimping members.

The supporting part is generally rectangular in shape and/or provides a spacing region of rectangular shape. The supporting part may be connected only to bottoms/bottom parts of the crimping members, whereby the branches of the crimping members may be free of any contact/connection with the tape/band of the assembly.

The supporting part is provided with a central aperture, optionally suitable for guiding the band assembly when a guiding pin is inserted in the central aperture, the guiding pin being provided for instance at a cutting plane where a cutting member extends to perform the cut. The guiding pin may act either as an abutment for the cutting member.

The band assembly may define a band assembly cutting site positioned at an intermediary position between the crimping members, which is a virtual separation line parallel to two adjacent straight edges of the crimping members when the crimping members have a U-shaped cross section (U-shaped profile).

Each crimping member is suitable to crimp locally the tube section, near a cutting site of the band assembly, provided the crimping member made of malleable and rigid material is crimped with the tube section already present in the receiving part. The flexible strip or band is connected to each crimping member and defines a cutting site at a junction region between two parts or halves of the band assembly, so that the band assembly can be cut at the cutting site that is shifted relative to any one of the crimping members.

A first crimping member is adjacent to the cutting site. When two crimping members are provided, the two crimping members are only separated by the junction region of the flexible strip or band in the band assembly. This allows the band assembly to be suitably prepositioned (around a tube section of a flexible tube to be cut), before cutting such tube section at location matching with/of the cutting site, provided the tube section is also clamped by the cutting device on either side of the tube region to be cut, for instance by a pair of tube holders included in the cutting device.

The cutting device is ready for a cutting when the band assembly coupled with the tube section is extending along elongation direction of the housing delimited by the tube holders. The band assembly may comprise a flexible band or tape made of resilient plastic (only one band, for instance), with two crimping members connected by this flexible band.

In embodiments of the band assembly, which is not claimed, one or more of the following features may be adopted:
- each of the crimping members has a pair of branches adapted (before the crimping) to be folded inwardly, while delimiting the receiving part for the flexible tube.
- the flexible strip or band may be thinner than any one of the two crimping members.
- the flexible strip or band overlaps the two crimping members and is provided with a central region arranged in the interspace.
- the flexible strip or band is adapted to be cut together with the flexible tube.
- material of each crimping member is magnetizable, allowing a magnetic coupling, which may be a magnet - crimping member coupling.
- the flexible band may be selectively attached to the two bottoms of the two crimping members (for instance without interfering with branches of the crimping member(s) adapted to be folded inwardly).
- the flexible band may have a width, possibly constant, that remains inferior to a width of the receiving part and/or inferior to width delimited at a lowermost surface of the housing (such lowermost surface being intended for contact with the band assembly).
- the band assembly comprises the two crimping members that are axially spaced and separated by a gap (as measured along direction of length of the crimping members).
- the strip is interconnecting the two crimping members and defines a junction portion, optionally made of a single layer of plastic material, that is cut simultaneously with the tube section.

In some options, the cutting device may be provided with a casing that is slotted or opened at two opposite sides and configured for having the flexible tube passing in two parallel passages such as slots or apertures, while also being movable along the two parallel passages. Such tube movement, obtained for the closed conformation of the tube holders, may be a sliding movement.
In particular embodiments, the casing may be provided with any suitable slot arrangement allowing the tube section to be guided inside the casing for the cut, while having the adjacent parts of the tube (external parts extending the tube section away from the housing) arranged outside the casing.

The cutting device may be provided with one or more of the following particular features:
- the casing may be provided with a guiding part for guiding the pair of tube holders, for instance to prevent any relative shifting between the tube holders.
- the first tube holder includes two first jaws with a lower first jaw and an upper first jaw, while the second tube holder includes two second jaws with a lower second jaw and an upper second jaw.
- at least one hinge connection connects the two first jaws together and the two second jaws together.
- the at least one hinge connection may have a hinge axis and is configured to slide inside the casing with said hinge axis movable linearly through the two parallel passages.
- the driving assembly comprises an actuating member movable relative to the casing, possibly parallel to the two parallel passages.
- the cutting member is arranged stationary in the casing, the tube section being cut in said interposed configuration of the band assembly in response to an action, which may be a pulling or pushing action, exerted on the actuating member.

Optionally, at least one pin is provided for sliding the tube holders relative to the casing, said pin being configured to allow displacement of the pair of tube holders before and/or when cutting the tube section by the cutting member, with the pin being engaged into a guiding path.
The guiding path may include:
- a first section so that the pin is displaced closer from a plane that passes through the two parallel slots, whereby the housing is decreasing in size to allow a crimping of the tube section received in the housing by the jaws;
- a second section (that is parallel to the two parallel slots, allowing the tube section already crimped to be displaced relative to the cutting member.

In some embodiments of the device, the guiding may be obtained using one or more of the following options:
- the guiding path maybe distributed in at least two regions, possibly four regions.
- a first pin, protruding from at least one amongst the first jaws, may be arranged to slide in a first sliding track or elongated slot provided in a wall of the casing.
- the first sliding track or elongated slot may have a straight part that extends parallel to the parallel passages, to form all or part of the second section.
- the straight part may guide a corresponding pin that is thus reaching the second section of the guiding path.
- a second pin, protruding from at least one amongst the second jaws, may be arranged to slide in a second sliding track or elongated slot provided in a wall of the casing.
- the second sliding track or elongated slot may have a straight part that extends parallel to the parallel slots to form all or part of the second section (such section may guide a corresponding pin that is thus reaching the second section of the guiding path).

According to another aspect, it is also provided a use of the cutting device of claim 1 with a band assembly to be coupled to a tube section around an outer surface thereof, to allow cutting of a flexible tube at the tube section by the cutting device as above defined, so that a cut end is obtained respectively at two tube portions of the flexible tube, which serves for circulation of a biopharmaceutical fluid, the cutting device being used to provide a disconnection when separating the two tube portions by:
- crimping a tube section of the flexible tube by crimping, by the first tube holder, a first crimping member of the band assembly suitable to locally surround a first one of the tube portions, while the other one of the tube portions is sealed via a crimping action involving the second tube holder (such crimping being performed in a coupled state in which the band assembly extends around a tube section of the flexible tube, the tube section resting for instance on a bottom surface of the band assembly which may include a junction portion that is part of a flexible material distinct from the first crimping member); and
- cutting, by the cutting member, the tube section selectively at a band assembly cutting site of the band assembly, which is adjacent to the first crimping member crimped by the first tube holder on the one hand and adjacent to the second tube holder on the other hand, without cutting the first crimping member that maintains the cut end of said first tube portion in a closed state.

Possibly, the second tube portion may be sealed, differently, possibly using heat or irradiation by an irradiation source. In some options, a crimping part is also placed in the second tube holder, allowing a similar or identical sealing for the tube portion.
In the coupled state, the tube section may rest for instance on a bottom surface of the band assembly, the bottom surface being provided in a flexible material piece distinct from the crimping members.

In a particular aspect, it is also provided a use of a cutting device according to claim 2 with a band assembly including two crimping members (the band assembly being also intended to be coupled to a tube section around an outer surface thereof), in order to allow cutting a flexible tube by the cutting device as above defined, so that a cut end is obtained respectively at two tube portions of the flexible tube, which serves for circulation of a biopharmaceutical fluid, wherein the cutting device is used to provide a sterile disconnection when separating the two tube portions by:
- in a coupled state in which the band assembly extends around a tube section of the flexible tube, crimping said tube section by simultaneously crimping, by the pair of tube holders, a first crimping member of the band assembly suitable to locally surround one of the two tube portions and a second crimping member of the band assembly suitable to locally surround the other one of the two tube portions; and
- cutting, by the cutting member, the tube section selectively at the interspace between the tube holders of the cutting device without cutting the crimping members that maintain each of the two cut ends of the tube section in a closed state.

In the coupled state, the tube section may rest for instance on a bottom surface of the band assembly, the bottom surface having part thereof, possibly a central region of the bottom surface, included in a junction portion that is part of a flexible material/flexible material piece distinct from the crimping members.

In some embodiments, the band assembly has two halves adapted to be separated due to the cutting by the cutting member, the two halves being each marked for making each of the two tube portions traceable together with the corresponding band assembly half.

A flattened/crimped crimping member may also be present for connecting the band assembly half to the tube portion while also ensuring keeping a sealed state of the cut end, without involving heat/without using a heated blade.

According to a further aspect, which is not claimed, it is provided a method for obtaining a cutting device suitable to prepare and allow a flexible tube disconnection, the method comprising:
- providing at least one cutting member and a band assembly;
- arranging the band assembly in a housing delimited by a pair of tube holders, along a direction, the tube holders being spaced along the direction;
- maintaining a position of the band assembly inside the housing, in which positioning members interact with one or two crimping members of the band assembly, so that a band assembly cutting site is already predefined for said position;
- providing a driving assembly for driving the pair of tube holders from an open conformation, allowing installation of a tube section of the flexible tube in the housing, with the band assembly already in said position to have the tube section received between branches of the one or two crimping members, to a closed conformation, in which the tube holders are crimping said one or two crimping members and allowing a pinching of the tube section;
wherein an interspace separating a first holding region associated with the first tube holder from a second holding region associated with the second tube holder is suitable for allowing the flexible tube in installed state to be cut by the cutting member in the interspace with a simultaneous cut of the band assembly selectively at the band assembly cutting site without cutting any crimping member.

With such a method, the crimping may be performed efficiently, and a single operation may be involved for the crimping and the cutting, since the interspace between the tube holders provides suitable access for a cutting member.

Typically, the tube is flexible, forming a collapsible tube to allow sealing a lumen of the tube. The method may apply to a tube having a single lumen or to a double lumen tube.

The band assembly can be provided and mounted as a single block in the housing. A positioning step for installing and maintaining the band assembly is performed in open state of the tube holders. The positioning members may have two abutment parts in each lower jaw or in a similar lower delimitation part for delimitation of the housing, allowing a centering of the tube section that can extend only parallel to said direction (along which the tube holders are spaced). The positioning step may further involve at least one magnetic member. In some option, at least tone of the crimping member may include a magnet or may be provisionally coupled to a magnet unit.

The positioning step thus corresponds to a holding action where the tube section is prevented from slipping along said direction, while gravity and possibly magnetic attraction by a magnet act to prevent any upward displacement of the tube section with respect to bottom(s) or other suitable resting area formed in the crimping member(s).

### BRIEF DESCRIPTION OF THE DRAWINGS

The figures of the drawings are now briefly described.
Fig. 1 schematically illustrates a cutting device provided with a band assembly, for allowing, in a single operation, a crimping of two adjacent areas of a flexible tube section and a cutting at an intermediate space between two crimping sites.
Fig. 2A schematically illustrates a way of driving tube holders, here in an open conformation, with a predetermined guiding, so that the tube holders can reach a closed conformation in a crimping part of the device.
Fig. 2B is a cut view, similar to Fig. 2A, illustrating the closed conformation of the tube holders.
Fig. 3A is a view showing a rear face of a band assembly according to a first embodiment the band assembly having a site for the cutting and being suitable for an operation of crimping and disconnecting a flexible tube.
Fig. 3B is a view of a band assembly that is identical of similar to the assembly of Fig. 3A, with a receiving part shown delimited between branches of two crimping members included in the band assembly.
Fig. 3C illustrates a slight variant as compared to the band assembly of Fig. 3A, with an opening provided in a junction portion of a flexible band that joins the two crimping members.
Fig. 3D illustrates another variant for the band assembly.
Fig. 4 shows schematically a front view of the tube holders of a cutting device in a transitory state between a fully open conformation and a closed conformation, with a band assembly already associated with the tube section to be crimped and cut.
Fig. 5 illustrates the tube section of the flexible tube combined with two band assembly halves, after the cutting in accordance with an embodiment of the band assembly.
Fig. 6 shows five exemplary pieces involved to obtain a pair of tube holders suitable to allow a crimping effect, in an embodiment where a rod or hollow cylindrical shaft can form a part of a driving assembly.
Fig. 7 is a perspective view of an exemplary base part of the cutting device, in which a band assembly and the tube section have been successively mounted in a suitable centered position.
Fig. 8 shows a view of a receiving part of a cutting device before actuating a crimping and cutting operation, in an option where two crimping devices are placed differently, with one of them already secured to a first region of the tube section, while the other one is part of the band assembly and can extend around a second region (not shown) of the tube section.

### MORE DETAILED DESCRIPTION

A detailed description of several embodiments of the invention is provided below, accompanied with examples and with reference to the drawings.

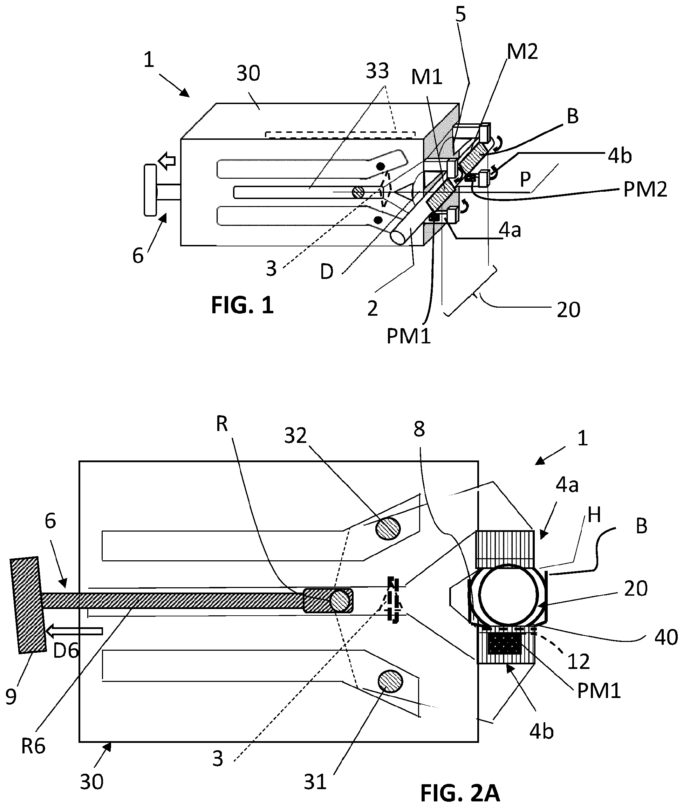

In the various figures, the same references are used to designate identical or similar elements. Some size or thickness may be exaggerated for the purpose of better illustration. For instance, spacing between the tube holders is exaggerated in Fig. 1 for the purpose of facilitating illustration of some functions and elements.

The embodiments described provide examples and should not be interpreted as limiting the scope of invention, which is defined by the claims.

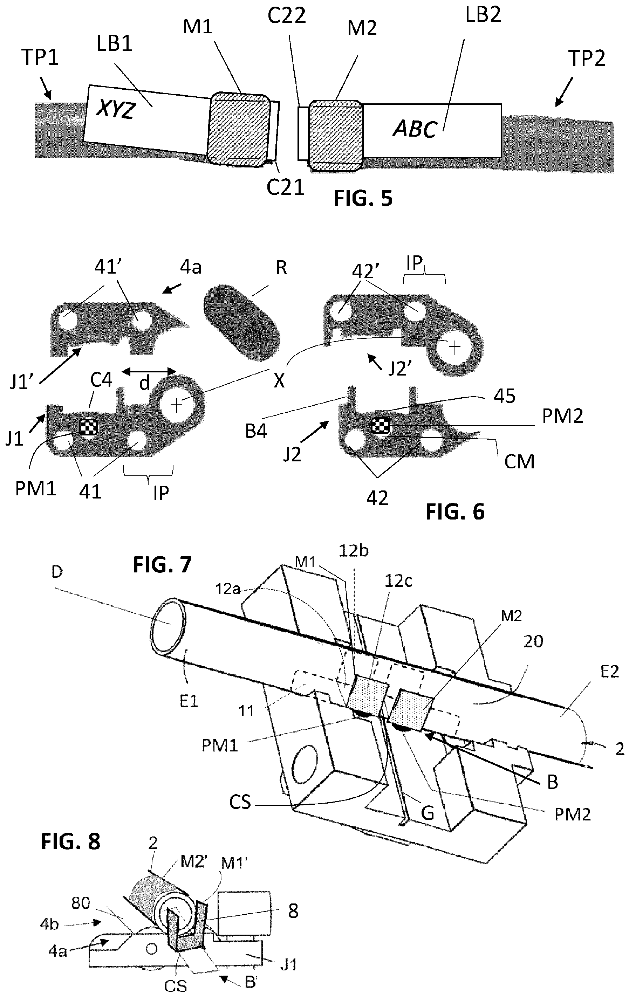

Referring to Figs 1 and 7, a cutting device 1 is including a holding part for receiving a flexible tube 2 that may be used in biopharmaceutical field. A cutting member 3 can be included in or coupled/associated to such cutting device 1, typically to allow performing a cut along a cutting plane that is perpendicular to an elongation direction of the flexible tube housed in the holding part. The cutting device 1 is provided with a holding part that is adapted to perform a crimping at a tube section 20 of the flexible tube 2, by passing from an open conformation (as illustrated schematically in Fig. 1 for instance) to a closed conformation.

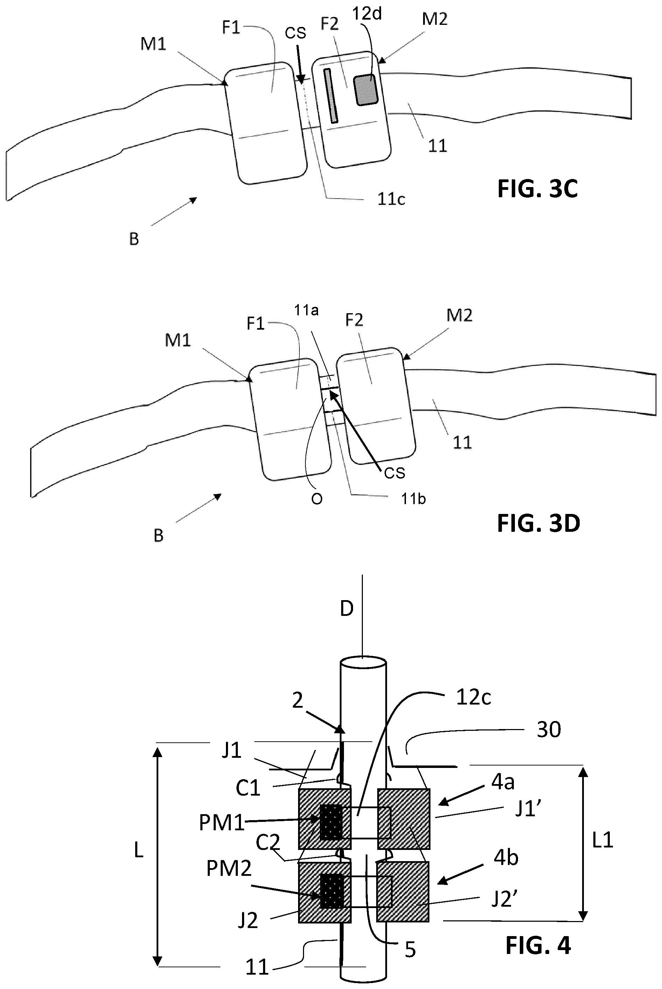

The holding part, provided with a pair of tube holders 4a, 4b, may have a transitory conformation such as illustrated in Fig. 4, when a driving assembly (or a user action) has started to obtain locally a pinching action, until obtaining a sealed state of the flexible tube 2 at two areas of the tube sections, respectively corresponding to two holding regions C1, C2. The cutting device 1 is configured to provide an interspace 5 located between the first tube holder 4a and the second tube holder 4b, such interspace 5 being well apparent in Fig. 4 for instance. The tube holders 4a, 4b may be provided with jaws J1, J1, J2, J2' (as for instance in Fig. 4 or 6) and, in the open conformation, they delimit a housing H easily accessible from a front side. The interspace 5 may include a central region of the housing H. It is understood the housing H is adapted for receiving the tube section 20 of the flexible tube to be cut (i.e. without any cut end when installing the tube section in the housing H).

The cutting device 1 may include a casing 30, inside which the tube holders 4a, 4b may extend in a closed conformation. For ease of installing the tube section 20 in the housing H, the tube holders 4a, 4b may extend outside the casing 30 to thus define an external housing H. A front recess R30 in the casing 30, which may be provided with a flared open end. A part of the tube holders 4a, 4b this is in an overlapping configuration with the cutting member 3 may extend at an interface region of the casing that delimits a rear delimitation of the front recess R30.

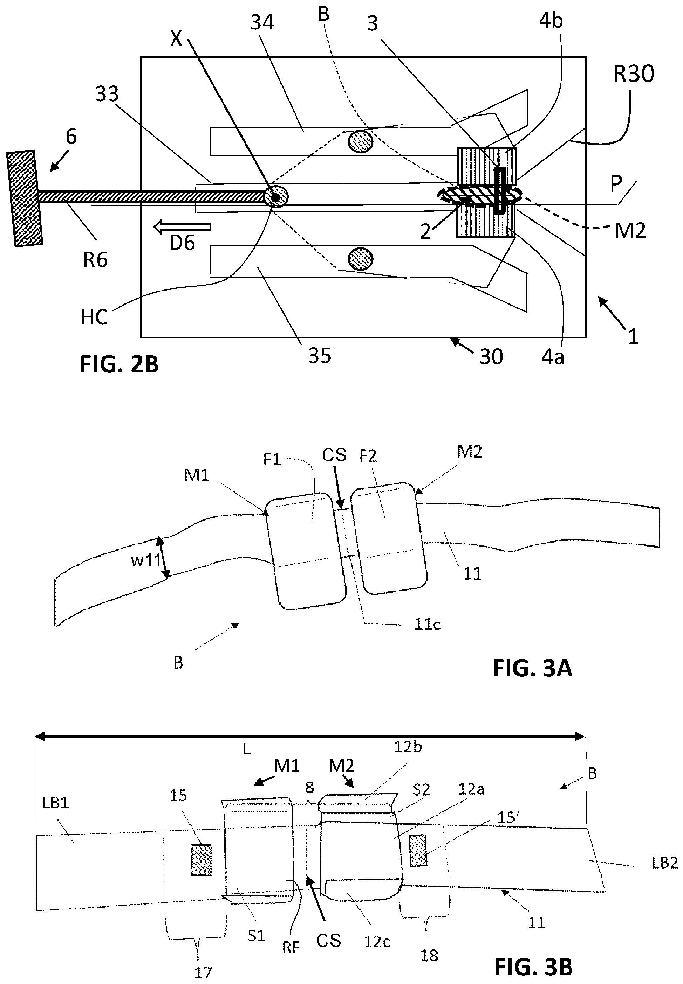

Referring to Figs 1, 2A and 2B, the driving assembly 6 may have a rod R6 or similar actuation part provided with a graspable actuating member 9 or similar handle part. A pulling action, along the pulling direction D6, may be exerted at such actuating member 9, in order to displace the tube holders 4a, 4b rearwardly, for instance toward a location inside an inner volume of the casing 30. The housing H is distributed, along a direction D, into two subhousings each delimited by one of the tube holders 4a or 4b. Here, there is no partition for such housing H but the cutting member 3 may extend as a (temporary) partition in the housing during actuation of the driving assembly 6.

The pair of tube holders 4a, 4b is arranged substantially in alignment and spaced along the direction D that may be considered as an axial direction for the housing H, so that in open conformation of the tube holders 4a, 4b, a radial access is provided for placing suitable elongated pieces to be cut. As reflected by Fig. 1, 4 or Fig. 8, the housing H is suitable for accommodation of a band assembly B that extends along the direction D and defines a receiving part for the tube section 20 of the flexible tube 2. An operator can either place the tube section 20 in the receiving part 8 such as shown in Fig. 3B, before inserting the tube section 20 together with the band assembly B in the housing H, or firstly dispose the band assembly B in the housing H and then place the tube section 20 in the receiving part 8.
In Fig. 1, the band assembly B and the flexible tube 2 are coupled, these elements being not yet resting on a lower part of the tube holders 4a, 4b. Of course, using lower jaws J1, J2, the band assembly B may also be preinstalled in lower cavity C4, with an upper access for the receiving part 8 still available.

In any one of such options for installation of the tube section 20 in the housing H, a flexibility is given for the location of the cutting position along length of the flexible tube 25. The cutting device 1 may comprise positioning means PM1, PM2 for maintaining the band assembly B inside the housing H, in an interposed configuration. The band assembly B is covering, in such interposed configuration, the tube section 20 that extends in the receiving part 8. The band assembly B is for instance covering the tube section 20 with:
- a covering from below and,
- a side coverage using rigid branches 12b, 12c (of the band assembly B) which serve for a crimping action, on either side of the tube section 20, typically with an interruption of such side coverage by the relatively rigid branches at location of the cut.

The cutting member 3 is configured in the cutting device 1 so that the interspace 5 defines a cutting plane. When positioning the band assembly B thanks to the positioning means PM1, PM2, a cutting site CS already present in the band assembly B is placed in the interspace 5. The branches 12b, 12c are configured to not interfere with the interspace 5, thus not being along trajectory of the blade or similar cutting member 3. When having jaws J1, J1', J2, J2' distributed in the structure of the tube holders 4a, 4b, on either side of the interspace 5, it is understood the tube section 20 can be cut by the cutting member 3 that is placed between the jaws J1, J1' and J2, J2'.

As well apparent in Figs 2A-2B, a crimping of parts of the band assembly B is allowed in such interposed configuration, while the flexible tube 2 is locally pinched by action of the tube holders 4a, 4b, whereby a lumen is closed in the flexible tube 2 at either side of the cutting site CS. Use of crimping members is of interest to maintain such closed/sealed state once the cut has been performed. In the interposed configuration, the band assembly B is contacting at least one amongst the first tube holder 4a and the second tube holder 4b. One or more crimping members M1, M2, M1' are part of the band assembly B and are deformable in response to a clamping action, so that the band assembly B locally surrounds the flexible tube with a crimping effect. Moreover, each crimping member can be disposed adjacent to the cutting site CS, to prevent any cutting contact between the cutting member 3 and such crimping member(s).

Some abutments may be provided to delimit a bottom surface of lower jaws J1, J2 of the tube holders, the band assembly B being resting on the bottom surface at a rear of the abutments. A lower cavity C4 may be provided with a front delimitation and possibly a rear delimitation to limit or block displacement of the band assembly B. An exemplary abutment projection or tooth B4 is illustrated in Fig. 6, along a side (open side) of the tube holders 4a, 4b which is wide open only in open conformation.

Fig. 6 provides an example of lower jaws J1, J2 and upper jaws J1', J2' that may serve to permit and clamping with pinching effect. A roll-like shaft or cylinder R may be made integral with a lower jaw J2 of the tube holder 4a and an upper jaw J1' of the tube holder 4b, to define a common axis X of the tube holders 4a, 4b. Such axis X is arranged at the opposite from the open side /mouth of the tube holder arrangement. The cylinder R or similar hinge connection of the pair of jaws J1, J1' and J2, J2' may be secured or connected to the driving assembly 6, as reflected in Fig. 2A for instance. A sliding movement of the hinge connection may be guided using one or more slots 33 or passages that may be provided in the casing 30. As illustrated in Fig. 2B for instance, the casing 30 may allow the flexible tube 2 passing in two parallel passages/slots 33 during movement of the driving assembly 6 to permit a cut at the interspace 5 between the first and second tube holders 4a, 4b, while distal tube parts E1, E2 may extend outside the casing 30. Such passage(s) may also serve for a guiding of at least one end the cylinder R or similar end provided at the hinge connection allowing the tube holders 4a, 4b passing from the open conformation (possibly an external conformation with respect to the casing 30) to the closed conformation, in which the cutting is or has been performed.

The cutting member 3 may be stationary in the casing 30 of the cutting device 1, for instance with the cutting member 3 being arranged between the axis X and the housing H. A spacing distance d along a front-rear direction (perpendicular to direction D) may thus be provided between the cavity C4 and the axis X, using jaws J1, J1', J2, J2' that have each an intermediate portion IP at the rear of the housing H, allowing the axis X to be offset rearwardly. When starting the crimping, the cutting member 3 may extend between the intermediate portions IP of the respective tube holders 4a and 4b, to allow starting the cutting at a rear of the flexible tube section (at the opposite from the mouth side).

In other options, the cutting member 3 may be movable. For instance, it may be moved with a counter-movement relative to displacement of the tube holders 4a, 4b, using an additional driving transmission or additional actuator, or using a displacement along a transverse direction with respect to a plane P that includes direction D and direction D6, while intersecting the flexible tube 2 held in the housing H.

Preferably, the housing H may decrease in size to allow a crimping of the tube section 20 received in the housing H, by the jaws J1, J1', J2, J2', while the tube section 20 may remain parallel to direction D and possibly without displacing the tube section with respect to plane P such as illustrated in Fig. 1. The plane P may extend along a front-rear direction. Such plane P may pass through two of the slots 33 or passages of the casing 30 when the casing is compact and of low width (width measured along direction D), possibly matching with extension of the tube holders 4a, 4b along the direction D.

### Exemplary guiding when crimping the flexible tube

When having a driving assembly 6 actuatable by a single action, for instance a pulling action, a guiding of the tube holders 4a, 4b may be provided. Also, movement of the jaws or mobile parts of the tube holders 4a, 4b may be synchronized, for instance using links. Referring to Fig. 6, it can be seen some pieces distributed in the tube holders 4a, 4b may be mutually fastened, using struts or linking elements. For instance, two lower jaws J1, J2 can be coupled using fastening means 41, 42, while two upper jaws J1', J2' can be coupled using other fastening means 41', 42'. Such linking elements may extend parallel to a hinge connection axis X (using a cylinder R or other hinging structure).

With such functional coupling of the tube holders 4a, 4b, the crimping effect at the first tube holder 4a and at the second tube holder 4b may be similar or identical, with a synchronizing effect. Referring to Figs 2A-2B, the driving assembly 6 may be quite simple, possible without involving a rotation to displace a common hinge connection established to allow the tube holder 4a, 4b to have a change in conformation. Rotation may be used only for jaws J1, J1', J2, J2' of the device 1. In such option, a lower part of the tube holders 4a, 4b may be provided with a lower pin 31, engaged through a slot of the casing 30, for guiding a sliding of the corresponding jaws J1, J2, while an upper part of the tube holders 4a ,4b may be provided with an upper pin 32, engaged through another slot of the casing 30, for guiding a sliding of the corresponding jaws J1', J2' (that extend above the mouth providing the front access for the housing H). The guiding slots may be parallel, except at respective ends away/ at the opposite from an actuating member 9 or end part of the driving assembly 6, with a divergence to increase a spacing between the pins 31 and 32 for a non-pulled state of the driving assembly 6, whereby the tube holders 4a, 4b can be initially in the open conformation allowing access to the housing H.

More generally, at least one pin 31, 32 or similar guiding member may be provided for sliding the tube holders 4a, 4b relative to the casing 30. Each pin may be configured to allow displacement of the pair of tube holders 4a, 4b before and/or when cutting the tube section 20 by the cutting member 3, possibly with each pin 31, 32 engaged into a guiding path (34, 35). In the non-limiting embodiment of Figs 2A-2B, such guiding path includes:
- a first section 30a so that the pin 31, 32 is displaced (progressively) closer from a plane P that passes through the two parallel slots or passages 33 for the tube 2, whereby the housing H is decreasing in size to allow a crimping of the tube section 20 received in the housing H by the jaws J1, J1', J2, J2';
- a second section 30b that is parallel to the two parallel slots or passages 33, allowing the tube section 20 already crimped to be displaced relative to the cutting member 3.

Of course, the crimping action can also be performed with another kind of driving assembly, for instance using a rotation for only one amongst the upper and the lower part of the tube holders 4a, 4b.

In any case, a crimping member integral with a flexible band 11 forming at least one junction portion 11a, 11b, 11 is involved to be cut in the housing H, simultaneously with the tube section 20, without cutting the crimping member(s) placed in the housing H so that the tubing part is closed in area adjacent to the cutting site CS. The cutting site CS in the band assembly B may be arranged to contact the tube 2 from below in the housing H and/or at a housing side where a magnetic attraction is involved with respect to one or more outer faces F1, F2 of the crimping members M1, M2.

When having a casing 30 provided with a suitable guiding part for guiding the pair of tube holders 4a, 4b, which may include a hinge connection HC for allowing movement of respective jaws J1, J1', J2, J2', a common axis X may facilitate actuation of the tube holders 4a, 4b for the crimping on the one hand, while possibly also facilitating the cutting when such axis X may be displaced using a sliding. When having a pullable driving assembly 6, the jaws J1, J1', J2, J2' provided at a head of the cutting device 1 can be pulled back and therefore are getting closed. This will crimp each branched crimping member M1, M2, M1', M2' (typically U-section metal crimping member) on the tube section 20 of the tubing and will properly seal the flexible tube 2.

### Examples of a band assembly

Referring to Figs 3A-3D, a band assembly B is shown with two crimping members M1, M2 and a flexible band 11, possibly under form of a single tape of flexible material. More generally, the band assembly B is of interest to provide an efficient disconnection at the tube section 20, independently from the specific kind of cutting device 1 used for applying a cutting at an interspace 5 between two holding regions C1, C2. The band assembly B has a geometry allowing the crimping members M1, M2, arranged with a spacing and in alignment along a direction matching/coinciding with the direction D, so that it may extend around the tube section 20 (of annular shape).

In the illustrated embodiments of Figs 3A-3D, a first crimping member M1 and a second crimping member M2 (here substantially of same shape and size) are included in the assembly B, each possibly being made of a folded piece of a strip/sheet material, for instance a metal sheet. The band assembly B also includes a tape, band 11 or similar supporting part of flexible material, which is secured to the crimping member M1 and M2. The band 11is here an elongated piece, extending along a first direction, which coincides with:
- direction D once the band assembly is received in the housing H, and
- elongation direction of the flexible section 20, when the flexible section 20 extends in the housing H while being disposed in the receiving part 8.

More generally, the flexible material of the band 11 may be plastic or similar material less rigid than strip or sheet material of any one amongst the crimping members M1, M2, M1', M2'. The band assembly B has branches 12b, 12c or similar parts involved in a crimping operation to seal the tube end parts adjacent to the tube cutting area. Such branches 12b, 12c may be distributed in one or more crimping members M1, M2, M1'. In the non-limiting embodiments of Figs 3A-3D, there are at least four branches distributed in the pair of crimping members M1, M2 that are each U-shaped. The branches 12b ,12c are thus the branches of the 'U' as seen in a cross-section perpendicular to direction D

In Fig. 3B for instance, it can be seen the band assembly B has a length, along direction D, which is more than three times any of the sizes of the crimping members M1 or M2. More generally, such length L of the band assembly B may be superior to a length L1 of the housing H, as reflected in Fig. 5. Thus, the flexible tube 2 is pinched at a region of limited extension and, after cut, the crimping length for each separated tube portion TP1, TP2 may be less than 20 or 25 mm for instance.

The receiving part 8 may form a channel that is open at a top of the band assembly B, at the opposite from bottom parts 12a of the crimping members M1, M2. These bottom parts form a base of the U-section as viewed perpendicular to direction D. Two surfaces S1, S2 of the crimping members, which are interior surfaces (concave surfaces as shown in the illustrated embodiments), and an additional surface of a junction portion 11c that is interconnecting the crimping members delimit such channel-like receiving part 8. Since the two crimping members M1, M2 are axially spaced and separated by a gap (gap as considered along direction D), only the strip material or suitable flexible material of the band 11 needs to be cut when the cutting site CS is matching with the interspace 5. The band B may be flat at least along the crimping member bottoms 12a, so that the branches 12b, 12c may be free of any contact/connection with the band 11.

While the junction portion 11c is shown as having a width identical to a band width in Figs 3A-3C, it is understood, the width may vary for the flexible band 11. Possibly, two or more bridging arms 11a, 11b (see Fig. 3D) may form links between the crimping members M1, M2, thus forming a discontinuous junction portion 11c. In embodiments, material of the band 11 may extend planar and the supporting part or band 11 of the band assembly B can extend planar at least at a spacing region (junction portion) made of the flexible material. The junction portion 11c can be maintained in a stretched state (i.e. without any folds at the interspace 5), possibly using an aperture or opening O for provisionally hold the band assembly B in a centered position, using a complementary guide provided in the tube holders 4a, 4b or provided between the tube holders 4a, 4b.

Now referring to Figs 3B and 5, since the cutting site CS of the band assembly B is present at the interspace 5 where the cutting member 3 is performing the cutting step, a crimping member M1, M2 cannot be cut, and no sharp edge is formed due to a cut. This applies to embodiments using any kind of shape/structure for the crimping member.

Moreover, the tape or band 11 may serve to include functional components and/or identifying devices. As shown in Fig. 5, the band 11 or tape part of the band assembly B may be provided with one or two identifying devices. At least one identifying device is allowing identification of the tube portion TP1, TP2 that is cut and surrounded by a subdivided portion of the band assembly B. A label LB1, LB2 with a serial number may form all or part of the identifying device.

Referring to Fig. 3B, the identifying device 15, 15' may be under the form of a barcode or QR code. Possibly a distal portion away from the crimping member may be detached, while a shorter label region 17, 18 may be kept secured to the crimping member M1, M2.

In some options a RFID device may be included in or mounted on the band 11. The identifying device or marking label may be offset relative to the crimping member M1, M2, away from the cut end C21, C22 of the corresponding tube portion TP1, TP2, and possibly distant from an opposite free end of the label LB1, LB2.

### Example of flexible band bonding two crimping members

The band assembly B may entirely consist of pieces that are relatively thin, for instance without any piece thicker than 3 or 4 mm. For instance, each crimping member M1, M2, M1' can be magnetizable and/or made of metal, with a thickness lower than 3 mm. Referring to Figs 3A-3D, the band 11 interconnecting the crimping members M1, M2 may be easy foldable with a maximal thickness inferior or equal to 2 mm. Accordingly, the spacing at the junction portion 11a, 11b, 11c may be inferior or equal to 2 mm. In some variants, a reinforcing strut or layer may be provided in the junction portion, to provide a stronger spacing effect, facilitating positioning of the band assembly in the housing H.
Besides, total length of the band assembly B may be defined by the band 11, which is for instance superior to 30 or 40 mm. Such length L may be superior to a length of the housing H and superior to two or three times the length of each crimping member M1, M2, M1' as measured along same direction D).

More generally, it is understood the supporting part material allows a bending, such material being flexible material or semi-rigid material (mainly flexible around a transverse axis). The flexible tape or band 11 may be transparent or translucent in some options. A marking may be provided to designate a cutting line corresponding to the cutting site CS.

In Figs 3A-3D, the band 11 serves to connect the two crimping members M1, M2 together. On both ends, the tape/strip/band 11 may be marked with a lot number or a serial number reflecting every connection. Such connection can be traced after disconnection. Alternatively, or in addition, such number or reference may possibly reflect a content of a bioreactor product contained in a single-use pouch or container in fluid communication with the flexible tube 2 at least before the cutting operation by the cutting device 1.

Regarding width w11 of the band 11, such w11 width may be reduced to be inferior or equal to an extension of each lower cavity C4 or similar lower delimitation in the housing H. Optionally, the width w11 is chosen substantially equal to extension of a lower face F1, F2 of the crimping members M1, M2, which may be a substantially planar face at least before the crimping.

While the band 11 may have a 2D design when the band assembly B is mounted in the housing H, it is understood each crimping member M1, M2, M1' has 3-D shape, with an axial extension or length along an elongation direction of the receiving part 8 (thus along direction D). Each crimping member M1, M2, M1' may also have a nominal width that may be superior or equal to width W11 of the strip/band 11. A transverse size or height of the crimping member M1, M2, M1' (typically corresponding to length of the branches 12b, 12c), forming a third size perpendicular to the band 11, is chosen to be higher than a nominal radius of the flexible tube 2. Such height may be adapted, depending on outer diameter of the flexible tube 2. In non-limiting examples, such height may be higher than width w11 and/or comprised between 8 and 24 mm.

The crimping member M1, M2, M1', is made of malleable material so that a final height of the band assembly B obtained after the crimping is much lower that the nominal size before crimping. Reduction in the third size of the band assembly B may be of at least 50% due to the crimping.

### Positioning of the band assembly

The band assembly B is compact and may be inserted in the housing H via a suitable access, such as mouth of the pair of tube holders 4a, 4 when holders 4a, 4b are arranged as an articulated clamp. More generally, positioning of the band assembly B may be performed before installation of the flexible section 20 in the housing H. To ensure the cutting site CS is provided at a predetermined location with respect to the band assembly B, the positioning means PM1, PM2 can cooperate with at least one portion of the band assembly B

Now referring to Figs 2A, 4 and 7, the cutting device 1 can be provided with positioning means PM1, PM2 that are mounted in the tube holders 4a, 4b, to guide and maintain a given position of the band assembly B. In some options, relief(s) and/or a magnetic force are involved. Preferably to facilitate installation, one or more of the crimping members M1, M2, M1,' M2' are magnetizable and can interact with magnet(s) or similar magnetic members provided in the positioning means PM1, PM2, which may extend along a face, for instance a bottom face, that delimits the housing H.

The outer faces F1, F2 of the crimping members M1, M2 may be relatively planar. More generally, such outer faces F1, F2 in the bottom parts 12a have a magnetic interaction with one or more magnetic member(s) forming the positioning means PM1, PM2. The magnetic members can be arranged in the lower jaws J1, J2, for instance in cavities CM, here arranged below a bottom of cavity C4, such as shown in Fig. 6.

It is understood the positioning members PM1, PM2 can also have abutments allowing a guiding and holding action. For instance, the positioning members PM1, PM2 are maintaining the band 11 of the band assembly B parallel to direction D and hold a contact configuration, in which a lower face of the band assembly is resting one the first tube holder 4a and typically also on the second tube holder 4b. Fig. 7 shows there is a centering effect, so that each crimping member M1, M2 remains close to/facing (at a bottom thereof in such example) a corresponding positioning member PM1, PM2. In some options, at least one relief 12d is provided in one or more crimping members M1, M2 for allowing a suitable positioning of the band assembly B. The relief(s) 12d may be provided for instance at one or more of the faces F1, F2 of the crimping members, which are faces turned at the opposite from the tube section 20 emplacement. More generally, at least one the positioning means PM1, PM2 can include a complementary relief(s), possibly included in a bottom part of a tube holder 4a or 4b, allowing a mechanical fastening or a coupling using shape complementarity with relief(s) 12d or with any discontinuity or opening O found in the band 11.

With help of such positioning members, the tube holders 4a, 4b are receiving the band assembly cutting site CS at the interspace 5, i.e. at an intermediary position between the first tube holder 4a and the second tube holder 4b. A groove G may also be provided at such interspace 5 (as illustrated in Fig. 7) for a guiding of cutting edge of the cutting member 3, for instance when a lower or upper part the tube holders 4a, 4b is formed as a single block as in the option of Fig. 7 (here a lower part).

When a lower part of the holding unit is designed a single block, the upper jaw portion (not shown for the case of Fig. 7) may include one or more pressing elements, forming lower protrusions with angled surfaces to allow a crimping effect. An interspace is provided to have a space between the two pressing regions where contact with the crimping members M1, M2 occurs, so that spaced tube holders are obtained. Locally flat surfaces are provided in the upper jaw portion on either side of an interspace 5 (interspace arranged in the upper jaw or separating two upper jaws that are synchronized in displacement). Such flat surfaces may contact the curved branches 12b, 12c at the end of the crimping or just before the end of the crimping. A cutting member 3 may be centered in the upper jaw portion, extending at the interspace 5.
In some variants, the cutting member may be displaced independently from displacement of any jaw portion, for instance when another actuation tool is available for allowing the cutting, possibly with actuation immediately after the crimping.

Whatever the structure chosen for jaws or similar movable parts involved for the crimping operation, the tube holders 4a, 4b may have an interspace 5 that is relatively narrow, allowing the cutting operation by the cutting member 3, the cutting operation causing the tube section 20, which is crimped at the respective holding regions C1, C2, to be cut transversally in coincidence with the band assembly cutting site CS without cutting a crimping member M1, M2, M1'.

The branches 12b, 12c of such crimping members M1, M2, M1' may be deformed to circumferentially surround the tube section 20, due to a progressive inward curving of the two branches 12b, 12c.

Referring to Fig. 5, a small protruding part of the separated tube portions TP1, TP2 may be present, beyond the respective crimping members M1, M2, and the cut ends C21, C22 may be provided at the cut ends C21, C22, with a sealed state due to the complete sealing of the tube portions TP1, TP2 in the crimped areas. An annular flattened conformation of the crimping members may be quickly obtained, to maintain a sealed state at each corresponding cut end C21 C22 of the flexible tube 2.

As shown in Figs 2A-2B, the tube holders 4a, 4b, can hold and progressively press the flexible tube section 20 around which one or more crimping members M1, M2, M1' of the band assembly B extend. In an operation mode of the cutting device 1, for suitable interposed configuration of the band assembly, the cutting through the interspace 5 may cause,
in a first option:
   - firstly, a cutting of the tube section 20, and
   - immediately after such tube cut, a cutting of the band assembly at the cutting site CS, knowing the cutting of the tube section 20 is typically performed perpendicular to the direction D and with the cutting member 3 entering the interspace 5 from a direction opposite to the cutting site;
or, in a second option:
   - simultaneously, a cutting of the tube section 20 and the cutting of the band 11 at the cutting site CS, provided a cutting edge of the cutting member 3 extends perpendicular to the cutting site CS of the band assembly during the cutting, as in embodiment of Figs 2A-2B.

The cutting device 1 is suitable to be actuated, possibly using a driving assembly 6 functionally coupled to the tube holders 4a, 4b, once the band assembly B has been mounted in the housing H. When having two crimping members M1, M2, the minimal spacing distance between the two crimping members M1, M2 (matching with the interspace just before a crimping) may be comprised between 1,5 and 10 mm.

### Tube

A tube 2 such as the one to which the invention applies makes possible the circulation, the passage, and the communication of a fluid, such as a biopharmaceutical fluid and such a tube may be typically interposed between two-or more-pouches. Such a tube, usually having a circular cross-section, is typically made of plastic material such as silicone, but also TPE or PVC, with the list not being limiting. Flexibles tubes 2 of the type Tuflux^{®} TPE or Tuflux^{®} SIL are exemplary tubes suitable to be cut, while being adapted for biopharmaceutical use.

It exhibits a certain overall behavior and, at the same time, both a certain flexibility as a whole and a certain local flexibility, which makes it possible, by means of exerting enough pinching force, to pinch the tube by flattening it and crushing it to close it on itself. Use of a crimping member M1, M2, M1' can also ensure the tube does not enlarge too much along direction D due to the crimping.
In a typical embodiment, for example, the tube 2 has an outside diameter of between, for example, 3 millimeters and 30 millimeters, whereby the thickness depends on material, diameter, and applications.

### Band assembly construction with two halves

When having two crimping members M1, M2, the band assembly B can have a two halves construction, with each half being heterogenous in composition: only the crimping member M1 or M2 contains metal with a rigidity that prevents accidental/unintentional deformation. In such non-limiting embodiment, the method of cutting the flexible tube 2 involves two metal pieces or similar malleable and rigid material pieces, distributed along length L of the band 11 of the band assembly. Such halves may be identical in size and in shape. Of course, the crimping members M1, M2 can be made separately, each of them having its own fixation region RF for securing the crimping member to the band 11.

Referring to Fig 3A-3B, the band assembly B has two halves adapted to be separated due to the cutting, with an identifying device 15, 15' distributed in each half. When the two tube portions TP1, TP2 are separated by the cutting member, they are already marked thanks to the fixing of the corresponding band assembly half, thus making such tub portion TP1 or TP2 traceable together with the corresponding identifying device 15, 15'.

Construction with symmetrical halves may be preferred. Accordingly, if fluid has to be swept from the cut end, a same sealing is performed on both sides of the cutting sites, provided the tube holders 4a, 4b have similar structure and operating mode for the crimping. The tube 2 can be efficiently closed and cut into sections in a given region so as to prevent the circulation, the passage, and the communication of fluid on either side of this region and to be able to have two separate tube portions after a cut, each being thus closed and sealed. Traceability is of interest as at least one amongst the tube portions TP1, TP2 may be still communicating (fluid communication) with an adjacent device, such as a flexible plastic pouch, a filter or other functional components.

Of course, the invention is not limited to the embodiments described above and provided only as examples. It encompasses the various modifications, alternative forms, and other variants conceivable to a skilled person within the context of the invention, and in particular any combinations of the various modes of operation described above, which may be taken separately or in combination.

For instance, while the device 1 includes tube holders or clamps adapted to hold the tube with use of a blade or similar cutting member 3 entering the interspace 5, two blades may be provided in a variant, forming first and second blades (parallel blades) that pass between the clamps. In such option, the first blade and second blade are provided at first and second workstations on the device. A guillotine like arrangement using one or more blades may be involved.

Also, while many drawings show a band assembly B with two crimping members M1, M2 already fastened to a common tape or supporting part such as flexible strip/band 11, a cutting operation may use a hybrid approach with an additional installation of a second crimping member M2' directly around the flexible tube, before mounting the modified tube section in the receiving cavity of a band assembly B' having only the first crimping member M1, with an offset between this first crimping member M1 and the crimping member already surrounding the tube. After an adjustment of the modified tube section, optionally by using the positioning means PM1, PM2 to ensure the modified tube section has its crimping member correctly placed (using for instance a magnet or similar second positioning means PM2).

Fig. 8 schematically shows a band assembly B extending between lower jaws of tube holders 4a, 4b and a flexible tube 2 (here partly illustrated to only show the tube section arranged on the tube holder 4b). Such band assembly B only has one crimping member M1' initially fastened to the tape or band that can be cut at a cutting site CS. The other crimping member M2', here a tubular member, may be associated to the tube 2 before arranging the section of the tube 2 in the housing of the tube holders 4a, 4b.

Once a positioning step is performed, possibly using a spacing between rollers 80 for positioning the tube section to be cut, with the crimping member M2' only separated from the crimping member M1' by a small interspace, a crimping operation may start. Such positioning with rollers 80 may be implied in other options than the one illustrated in Fig. 8, possibly with all or part of the rollers offset with respect to crimping area. Only lower jaw(s) J1 is illustrated in such exemplary embodiment.

When performing the crimping, a cutting is also performed simultaneously or after the crimping at the cutting site CS to also cut the flexible tube 2 selectively between the crimping member M1' of the band assembly B and the other crimping member M2'. It is noted a choice exists for location of the cut since the operator may choose to offset the cut with respect to either side of the crimping member M2'. Optionally, an adhesive part may be provided in the band 11 to allow a fastening of a half of the band 11 not having the crimping member M1' fastened to the crimping member M2', before actuating the tube holders for the crimping action. Typically, the half provided with the crimping member M1' allows a sealing of corresponding tube portion as efficient as in other previously described embodiments and no sharp edge exists, neither in the member M1', nor in the member M2.

## Claims

1. A cutting device (1) for cutting a flexible tube (2) at a tube section (20) thereof, the cutting device (1) comprising:
- at least one cutting member (3);
- a band assembly (B; B') comprising a first crimping member (M1; M1') adjacent to a band assembly cutting site (CS);
- a first tube holder (4a);
- a second tube holder (4b), the first tube holder and the second tube holder being a pair of tube holders adapted to delimit a housing (H) for receiving the tube section (20) of the flexible tube (2) to be cut;
- an interspace (5), located between the first tube holder (4a) and the second tube holder (4b), the interspace (5) separating a first holding region (C1) associated with the first tube holder (4a) from a second holding region (C2) associated with the second tube holder (4b); and
- a driving assembly (6) for driving the pair of tube holders (4a, 4b) from an open conformation, allowing installation of the tube section (20) in the housing (H), to a closed conformation, in which the first tube holder (4a) is crimping the first crimping member (M1) and participates in pinching the tube section (20) together with the second tube holder (4b);
wherein the band assembly (B; B') is adapted to delimit, before any crimping, a receiving part (8) at least partly delimited by a surface (S1) of the first crimping member (M1; M1') for accommodation of the tube section (20); and
wherein the cutting device (1) comprises positioning means (PM1, PM2) that are configured to maintain the band assembly (B; B') inside the housing (H), in an interposed configuration for:
- covering the tube section (20) extending in the receiving part (8) on the one hand,
- and contacting at least one amongst the first tube holder (4a) and the second tube holder (4b) on the other hand, with the band assembly cutting site (CS) positioned at an intermediary position between the first tube holder (4a) and the second tube holder (4b),
whereby a cutting operation performed for said interposed configuration, by the cutting member (3) through the interspace (5), causes the tube section (20) to be cut transversally at the band assembly cutting site (CS) without cutting the first crimping member (M1; M1') that is crimped in the closed conformation.

2. The cutting device according to claim 1, wherein the band assembly (B) comprises two distinct crimping members, including the first crimping member (M1) and a second crimping member (M2),
wherein in the closed conformation, the first tube holder (4a) and the second tube holder (4b) are crimping the two crimping members (M1, M2) and pinching the tube section (20), wherein the receiving part (8) for accommodation of the tube section (20) is delimited by two interior surfaces (S1, S2), mutually spaced and preferably each generally concave, of the two crimping members (M1, M2), the two interior surfaces including the first surface (S1),
and wherein the positioning means (PM1, PM2) are configured to maintain inside the housing (H) the band assembly (B) in contact with the first tube holder (4a) and with the second tube holder (4b), while the band assembly (B) extends inside the receiving part (8), the positioning means (PM1, PM2) functionally cooperating with at least one positioning part of the band assembly, preferably with the two crimping members (M1, M2), to have the two crimping members (M1, M2) distributed respectively, preferably selectively distributed, in the first holding region (C1) and in the second holding region (C2);
whereby the cutting at the band assembly cutting site (CS) is a cutting performed in an intermediate region between the two crimping members (M1, M2) for also cutting the tube section (20) transversally without cutting any one of the two crimping members (M1, M2) that are crimped in the closed conformation of the pair of tube holders (4a, 4b).

3. The cutting device according to claim 2, wherein the positioning means (PM1, PM2) comprises two magnetic members adapted to magnetically interact with the two crimping members (M1, M2) each constituted of or including a magnetizable material,
and wherein the crimping members (M1, M2) are each made of a malleable and rigid composition.

4. The cutting device according to claim 2, wherein a minimal spacing distance between the two crimping members (M1, M2), preferably matching with the interspace (5) just before a crimping due to displacement toward the closed conformation, is comprised between 1,5 and 10 mm.
and wherein the band assembly (B) is a 3-D device with each of the crimping members (M1, M2) adapted to be crimped from an initial U-shaped conformation to an annular conformation that is flattened to maintain a sealed state at each corresponding cut end of the flexible tube (2).

5. The cutting device according to claim 3 or 4 when depending on claim 3, wherein the crimping members (M1, M2) are each defining a U-section with a bottom (12a), the two magnetic members being arranged on a same side of the housing (H) in the pair of tube holders (4a, 4b), in order to attract each bottom (12a) of the crimping members (M1, M2), along said same side before installation of the tube section (20) in the housing (H).

6. The cutting device according to claim 2, wherein the positioning means (PM1, PM2) comprises a magnetic member adapted to magnetically interact with at least one amongst the two crimping members (M1, M2),
and wherein the crimping members (M1, M2) are each made of a malleable and rigid composition.

7. The cutting device according to any of the preceding claims, wherein the pair of tube holders (4a, 4b) is arranged substantially in alignment and spaced along a first direction (D) in an operation mode of the cutting device (1), for said interposed configuration, so that the cutting operation performed by the cutting member (3) through the interspace (5) and/or at the band assembly cutting site (CS) is a cutting of the tube section (20) perpendicular to the first direction (D).

8. The cutting device according to claim 2 alone or combined with any one of claims 3-7, wherein the band assembly (B) comprises a flexible band (11), and wherein the two crimping members (M1, M2) are connected by the flexible band (11), preferably thinner than the two crimping members, which overlaps the two crimping members (M1, M2) and is provided with a central region arranged in the interspace (5) and adapted to be cut together with the flexible tube (2).

9. The cutting device according to claim 2 alone or combined with any one of claims 3-7, wherein the band assembly (B) comprises:
- the two crimping members (M1, M2) that are axially spaced and separated by a gap; and
- a strip provided with two identifying devices (15, 15'), the strip interconnecting the two crimping members (M1, M2) and defining a junction portion (11c) that is cut simultaneously with the tube section (20).

10. The cutting device according to any one of the preceding claims, comprising a casing (30) that is slotted or opened at two opposite sides and configured for having the flexible tube (2) passing in two parallel passages, preferably formed as slots (33), while also being movable along the two parallel passages.

11. The cutting device according to claim 10, wherein the casing (30) is provided with a guiding part for guiding the pair of tube holders, the first tube holder (4a) including two first jaws with a lower first jaw (J1) and an upper first jaw (J1'), while the second tube holder (4b) includes two second jaws with a lower second jaw (J2) and an upper second jaw (J2'),
wherein at least one hinge connection (HC) connects the two first jaws together and the two second jaws together,
and wherein the at least one hinge connection (HC) has a hinge axis (X) and is configured to slide inside the casing with said hinge axis (X) movable linearly through the two parallel passages.

12. The cutting device according to claim 10 or 11, wherein the driving assembly (6) comprises an actuating member (9) movable relative to the casing (30), parallel to the two parallel passages,
wherein the cutting member (3) is arranged stationary in the casing (30), the tube section (20) being cut in said interposed configuration of the band assembly (B) in response to a pulling or pushing action exerted on the actuating member (9);

13. The cutting device according to any one of claims 10-12, wherein at least one pin (31, 32) is provided for sliding the tube holders (4a, 4b) relative to the casing (30), said pin (31, 32) being configured to allow displacement of the pair of tube holders (4a, 4b) before and/or when cutting the tube section (20) by the cutting member (3), with the pin (31, 32) being engaged into a guiding path (34, 35), the guiding path including:
- a first section (30a) so that the pin (31, 32) is displaced closer from a plane (P) that passes through the two parallel slots (33), whereby the housing (H) is decreasing in size to allow a crimping of the tube section (20) received in the housing (H) by the jaws (J1, J1', J2, J2');
- a second section (30b) that is parallel to the two parallel slots (33), allowing the tube section (20) already crimped to be displaced relative to the cutting member (3).

14. The cutting device according to any one of the preceding claims, wherein the band assembly (B) has a length (L), which is a length of an elongated flexible strip or band (11), that is superior to a length (L1) of the housing (H).

15. The cutting device according to any one of the preceding claims, wherein each of the crimping members (M1, M2) is provided with protrusions or reliefs that cooperate mechanically, preferably without any adhesive interaction, with the tube holders (4a, 4b).

16. Use of the cutting device (1) according to claim 1, wherein the band assembly (B; B') is coupled to a tube section around an outer surface thereof to allow cutting a flexible tube (2) at the tube section by the cutting device (1), so that a cut end is obtained respectively at two tube portions (TP1, TP2) of the flexible tube (2), which serves for circulation of a biopharmaceutical fluid, wherein the cutting device (1) is used to provide a disconnection when separating the two tube portions (TP1, TP2) by:
- crimping a tube section (20) of the flexible tube by crimping, by the first tube holder (4a), a first crimping member (M1; M1') of the band assembly (B; B1') suitable to locally surround a first one of the tube portions (TP1), while the other one of the tube portions (TP2) is sealed via a crimping action involving the second tube holder (4b); and
- cutting, by the cutting member (3), the tube section (20) selectively at a band assembly cutting site (CS) of the band assembly, which is adjacent to the first crimping member (M1;
M1') crimped by the first tube holder (4a) on the one hand and adjacent to the second tube holder on the other hand, without cutting the first crimping member (M1; M1') that maintains the cut end of said first tube portion (TP1) in a closed state.

17. Use of the cutting device according to claim 2, wherein the band assembly (B) allows cutting a flexible tube (2) by the cutting device (1), so that a cut end is obtained respectively at two tube portions (TP1, TP2) of the flexible tube (2), which serves for circulation of a biopharmaceutical fluid, wherein the cutting device (1) is used to provide a sterile disconnection when separating the two tube portions (TP1, TP2) by:
- in a coupled state in which the band assembly (B) extends around a tube section (20) of the flexible tube (2), crimping said tube section (20) by simultaneously crimping, by the pair of tube holders (4a, 4b), a first crimping member (M1) of the band assembly (B) suitable to locally surround one of the two tube portions (TP1) and a second crimping member (M2) of the band assembly (B) suitable to locally surround the other one of the two tube portions (TP2); and
- cutting, by the cutting member (3), the tube section (20) selectively at the interspace (5) between the tube holders (4a, 4b) of the cutting device (1) without cutting the crimping members (M1, M2) that maintain each of the two cut ends of the tube section in a closed state.

18. Use according to claim 16 or 17, wherein the band assembly (B; B') has two halves adapted to be separated due to the cutting by the cutting member (3), the two halves being each marked for making each of the two tube portions (TP1, TP2) traceable together with the corresponding band assembly half.

## Patentansprüche

1. Schneidvorrichtung (1) zum Schneiden eines flexiblen Schlauchs (2) an einem Schlauchabschnitt (20) desselben, wobei die Schneidvorrichtung (1) umfasst:
- mindestens ein Schneidelement (3);
- eine Bandanordnung (B; B'), die ein erstes Verpresselement (M1; M1') benachbart zu einer Schneidstelle der Bandanordnung (CS) umfasst;
- einen ersten Schlauchhalter (4a);
- einen zweiten Schlauchhalter (4b), wobei der erste Schlauchhalter und der zweite Schlauchhalter ein Paar von Schlauchhaltern sind, die dazu geeignet sind, einen Aufnahmeraum (H) zum Aufnehmen des zu schneidenden Schlauchabschnitts (20) des flexiblen Schlauchs (2) zu begrenzen;
- einen Zwischenraum (5), der zwischen dem ersten Schlauchhalter (4a) und dem zweiten Schlauchhalter (4b) angeordnet ist, wobei der Zwischenraum (5) einen ersten Haltebereich (C1), der dem ersten Schlauchhalter (4a) zugeordnet ist, von einem zweiten Haltebereich (C2), der dem zweiten Schlauchhalter (4b) zugeordnet ist, trennt; und
- eine Antriebsanordnung (6) zum Antreiben des Paars von Schlauchhaltern (4a, 4b) aus einer offenen Konformation, die das Einbringen des Schlauchabschnitts (20) in den Aufnahmeraum (H) ermöglicht, in eine geschlossene Konformation, in der der erste Schlauchhalter (4a) das erste Verpresselement (M1) verpresst und gemeinsam mit dem zweiten Schlauchhalter (4b) am Quetschen des Schlauchabschnitts (20) beteiligt ist;
wobei die Bandanordnung (B; B') dazu ausgebildet ist, vor jeglichem Verpressen einen Aufnahmeteil (8) zu begrenzen, der zumindest teilweise durch eine Fläche (S1) des ersten Verpresselements (M1; M1') begrenzt ist, zur Aufnahme des Schlauchabschnitts (20); und
wobei die Schneidvorrichtung (1) Positioniermittel (PM1, PM2) umfasst, die dazu konfiguriert sind, die Bandanordnung (B; B') innerhalb des Aufnahmeraums (H) in einer zwischengeordneten Konfiguration zu halten, um:
- einerseits den Schlauchabschnitt (20), der sich in dem Aufnahmeteil (8) erstreckt, zu bedecken,
- und andererseits mit mindestens einem von dem ersten Schlauchhalter (4a) und dem zweiten Schlauchhalter (4b) in Kontakt zu treten, wobei die Schneidstelle der Bandanordnung (CS) an einer Zwischenposition zwischen dem ersten Schlauchhalter (4a) und dem zweiten Schlauchhalter (4b) positioniert ist,
wodurch ein für die zwischengeordnete Konfiguration durch das Schneidelement (3) durch den Zwischenraum (5) hindurch ausgeführter Schneidvorgang bewirkt, dass der Schlauchabschnitt (20) an der Schneidstelle der Bandanordnung (CS) quer geschnitten wird, ohne das erste Verpresselement (M1; M1') zu schneiden, das in der geschlossenen Konformation verpresst ist.

2. Schneidvorrichtung nach Anspruch 1, wobei die Bandanordnung (B) zwei verschiedene Verpresselemente umfasst, einschließlich des ersten Verpresselements (M1) und eines zweiten Verpresselements (M2),
wobei in der geschlossenen Konformation der erste Schlauchhalter (4a) und der zweite Schlauchhalter (4b) die zwei Verpresselemente (M1, M2) verpressen und den Schlauchabschnitt (20) quetschen,
wobei der Aufnahmeteil (8) zur Aufnahme des Schlauchabschnitts (20) durch zwei innere Flächen (S1, S2) begrenzt ist, die zueinander beabstandet und vorzugsweise jeweils im Allgemeinen konkav sind, der zwei Verpresselemente (M1, M2), wobei die zwei inneren Flächen die erste Fläche (S1) einschließen,
und wobei die Positioniermittel (PM1, PM2) dazu konfiguriert sind, innerhalb des Aufnahmeraums (H) die Bandanordnung (B) in Kontakt mit dem ersten Schlauchhalter (4a) und mit dem zweiten Schlauchhalter (4b) zu halten, während sich die Bandanordnung (B) innerhalb des Aufnahmeteils (8) erstreckt, wobei die Positioniermittel (PM1, PM2) funktionell mit mindestens einem Positionierteil der Bandanordnung zusammenwirken, vorzugsweise mit den zwei Verpresselementen (M1, M2), um die zwei Verpresselemente (M1, M2) jeweils, vorzugsweise selektiv, in dem ersten Haltebereich (C1) und in dem zweiten Haltebereich (C2) zu verteilen;
wodurch das Schneiden an der Schneidstelle der Bandanordnung (CS) ein Schneiden ist, das in einem Zwischenbereich zwischen den zwei Verpresselementen (M1, M2) ausgeführt wird, um auch den Schlauchabschnitt (20) quer zu schneiden, ohne irgendeines der zwei Verpresselemente (M1, M2) zu schneiden, die in der geschlossenen Konformation des Paars von Schlauchhaltern (4a, 4b) verpresst sind.

3. Schneidvorrichtung nach Anspruch 2, wobei die Positioniermittel (PM1, PM2) zwei Magnetelemente umfassen, die dazu geeignet sind, magnetisch mit den zwei Verpresselementen (M1, M2) zu wechselwirken, die jeweils aus einem magnetisierbaren Material bestehen oder ein solches einschließen,
und wobei die Verpresselemente (M1, M2) jeweils aus einer verformbaren und starren Zusammensetzung hergestellt sind.

4. Schneidvorrichtung nach Anspruch 2, wobei ein minimaler Abstand zwischen den zwei Verpresselementen (M1, M2), der vorzugsweise dem Zwischenraum (5) unmittelbar vor einem Verpressen aufgrund einer Verlagerung in Richtung der geschlossenen Konformation entspricht, zwischen 1,5 und 10 mm beträgt.
und wobei die Bandanordnung (B) eine 3-D-Vorrichtung ist, wobei jedes der Verpresselemente (M1, M2) dazu ausgebildet ist, aus einer anfänglichen U-förmigen Konformation in eine ringförmige Konformation verpresst zu werden, die abgeflacht ist, um an jedem entsprechenden Schnittende des flexiblen Schlauchs (2) einen abgedichteten Zustand aufrechtzuerhalten.

5. Schneidvorrichtung nach Anspruch 3 oder 4, wenn dieser von Anspruch 3 abhängt, wobei die Verpresselemente (M1, M2) jeweils einen U-Querschnitt mit einem Boden (12a) definieren, wobei die zwei Magnetelemente auf einer gleichen Seite des Aufnahmeraums (H) in dem Paar von Schlauchhaltern (4a, 4b) angeordnet sind, um jeden Boden (12a) der Verpresselemente (M1, M2) entlang der gleichen Seite anzuziehen, vor dem Einbringen des Schlauchabschnitts (20) in den Aufnahmeraum (H).

6. Schneidvorrichtung nach Anspruch 2, wobei die Positioniermittel (PM1, PM2) ein Magnetelement umfassen, das dazu geeignet ist, magnetisch mit mindestens einem von den zwei Verpresselementen (M1, M2) zu wechselwirken,
und wobei die Verpresselemente (M1, M2) jeweils aus einer verformbaren und starren Zusammensetzung hergestellt sind.

7. Schneidvorrichtung nach einem der vorhergehenden Ansprüche, wobei das Paar von Schlauchhaltern (4a, 4b) in einem Betriebsmodus der Schneidvorrichtung (1) für die zwischengeordnete Konfiguration im Wesentlichen fluchtend angeordnet und entlang einer ersten Richtung (D) beabstandet ist, sodass der durch das Schneidelement (3) durch den Zwischenraum (5) hindurch und/oder an der Schneidstelle der Bandanordnung (CS) ausgeführte Schneidvorgang ein Schneiden des Schlauchabschnitts (20) senkrecht zur ersten Richtung (D) ist.

8. Schneidvorrichtung nach Anspruch 2 allein oder in Kombination mit einem der Ansprüche 3 bis 7, wobei die Bandanordnung (B) ein flexibles Band (11) umfasst, und wobei die zwei Verpresselemente (M1, M2) durch das flexible Band (11) verbunden sind, das vorzugsweise dünner als die zwei Verpresselemente ist, das die zwei Verpresselemente (M1, M2) überlappt und mit einem zentralen Bereich versehen ist, der in dem Zwischenraum (5) angeordnet und dazu ausgebildet ist, gemeinsam mit dem flexiblen Schlauch (2) geschnitten zu werden.

9. Schneidvorrichtung nach Anspruch 2 allein oder in Kombination mit einem der Ansprüche 3 bis 7, wobei die Bandanordnung (B) umfasst:
- die zwei Verpresselemente (M1, M2), die axial beabstandet und durch einen Spalt getrennt sind; und
- einen Streifen, der mit zwei Identifizierungseinrichtungen (15, 15') versehen ist, wobei der Streifen die zwei Verpresselemente (M1, M2) miteinander verbindet und einen Verbindungsabschnitt (11c) definiert, der gleichzeitig mit dem Schlauchabschnitt (20) geschnitten wird.

10. Schneidvorrichtung nach einem der vorhergehenden Ansprüche, umfassend ein Gehäuse (30), das an zwei gegenüberliegenden Seiten geschlitzt oder geöffnet und dazu konfiguriert ist, den flexiblen Schlauch (2) durch zwei parallele Durchgänge, die vorzugsweise als Schlitze (33) ausgebildet sind, hindurchzuführen, während es außerdem entlang der zwei parallelen Durchgänge bewegbar ist.

11. Schneidvorrichtung nach Anspruch 10, wobei das Gehäuse (30) mit einem Führungsteil zum Führen des Paars von Schlauchhaltern versehen ist, wobei der erste Schlauchhalter (4a) zwei erste Backen mit einer unteren ersten Backe (J1) und einer oberen ersten Backe (J1') einschließt, während der zweite Schlauchhalter (4b) zwei zweite Backen mit einer unteren zweiten Backe (J2) und einer oberen zweiten Backe (J2') einschließt,
wobei mindestens eine Gelenkverbindung (HC) die zwei ersten Backen miteinander und die zwei zweiten Backen miteinander verbindet,
und wobei die mindestens eine Gelenkverbindung (HC) eine Gelenkachse (X) aufweist und dazu konfiguriert ist, innerhalb des Gehäuses zu gleiten, wobei die Gelenkachse (X) linear durch die zwei parallelen Durchgänge bewegbar ist.

12. Schneidvorrichtung nach Anspruch 10 oder 11, wobei die Antriebsanordnung (6) ein Betätigungselement (9) umfasst, das relativ zum Gehäuse (30) bewegbar ist, parallel zu den zwei parallelen Durchgängen,
wobei das Schneidelement (3) feststehend in dem Gehäuse (30) angeordnet ist, wobei der Schlauchabschnitt (20) in der zwischengeordneten Konfiguration der Bandanordnung (B) als Reaktion auf eine auf das Betätigungselement (9) ausgeübte Zug- oder Druckwirkung geschnitten wird;

13. Schneidvorrichtung nach einem der Ansprüche 10 bis 12, wobei mindestens ein Stift (31, 32) zum Verschieben der Schlauchhalter (4a, 4b) relativ zum Gehäuse (30) vorgesehen ist, wobei der Stift (31, 32) dazu konfiguriert ist, eine Verlagerung des Paars von Schlauchhaltern (4a, 4b) vor und/oder beim Schneiden des Schlauchabschnitts (20) durch das Schneidelement (3) zu ermöglichen, wobei der Stift (31, 32) in eine Führungsbahn (34, 35) eingreift, wobei die Führungsbahn umfasst:
- einen ersten Abschnitt (30a), sodass der Stift (31, 32) näher an eine Ebene (P) verlagert wird, die durch die zwei parallelen Schlitze (33) verläuft, wodurch der Aufnahmeraum (H) an Größe abnimmt, um ein Verpressen des in dem Aufnahmeraum (H) aufgenommenen Schlauchabschnitts (20) durch die Backen (J1, J1', J2, J2') zu ermöglichen;
- einen zweiten Abschnitt (30b), der parallel zu den zwei parallelen Schlitzen (33) ist und es ermöglicht, dass der bereits verpresste Schlauchabschnitt (20) relativ zum Schneidelement (3) verlagert wird.

14. Schneidvorrichtung nach einem der vorhergehenden Ansprüche, wobei die Bandanordnung (B) eine Länge (L) aufweist, die eine Länge eines langgestreckten flexiblen Streifens oder Bands (11) ist, die größer als eine Länge (L1) des Aufnahmeraums (H) ist.

15. Schneidvorrichtung nach einem der vorhergehenden Ansprüche, wobei jedes der Verpresselemente (M1, M2) mit Vorsprüngen oder Reliefs versehen ist, die mechanisch, vorzugsweise ohne jegliche adhäsive Wechselwirkung, mit den Schlauchhaltern (4a, 4b) zusammenwirken.

16. Verwendung der Schneidvorrichtung (1) nach Anspruch 1, wobei die Bandanordnung (B; B') um eine Außenfläche eines Schlauchabschnitts herum mit diesem gekoppelt ist, um ein Schneiden eines flexiblen Schlauchs (2) an dem Schlauchabschnitt durch die Schneidvorrichtung (1) zu ermöglichen, sodass ein Schnittende jeweils an zwei Schlauchteilen (TP1, TP2) des flexiblen Schlauchs (2), der zur Zirkulation eines biopharmazeutischen Fluids dient, erhalten wird, wobei die Schneidvorrichtung (1) verwendet wird, um beim Trennen der zwei Schlauchteile (TP1, TP2) eine Trennung bereitzustellen durch:
- Verpressen eines Schlauchabschnitts (20) des flexiblen Schlauchs durch Verpressen, durch den ersten Schlauchhalter (4a), eines ersten Verpresselements (M1; M1') der Bandanordnung (B; B1'), das dazu geeignet ist, ein erstes der Schlauchteile (TP1) örtlich zu umgeben, während das andere der Schlauchteile (TP2) über einen den zweiten Schlauchhalter (4b) einbeziehenden Verpressvorgang abgedichtet wird; und
- Schneiden, durch das Schneidelement (3), des Schlauchabschnitts (20) selektiv an einer Schneidstelle der Bandanordnung (CS) der Bandanordnung, die einerseits benachbart zu dem durch den ersten Schlauchhalter (4a) verpressten ersten Verpresselement (M1; M1') und andererseits benachbart zu dem zweiten Schlauchhalter ist, ohne das erste Verpresselement (M1; M1') zu schneiden, das das Schnittende des ersten Schlauchteils (TP1) in einem geschlossenen Zustand hält.

17. Verwendung der Schneidvorrichtung nach Anspruch 2, wobei die Bandanordnung (B) ein Schneiden eines flexiblen Schlauchs (2) durch die Schneidvorrichtung (1) ermöglicht, sodass ein Schnittende jeweils an zwei Schlauchteilen (TP1, TP2) des flexiblen Schlauchs (2), der zur Zirkulation eines biopharmazeutischen Fluids dient, erhalten wird, wobei die Schneidvorrichtung (1) verwendet wird, um beim Trennen der zwei Schlauchteile (TP1, TP2) eine sterile Trennung bereitzustellen durch:
- in einem gekoppelten Zustand, in dem sich die Bandanordnung (B) um einen Schlauchabschnitt (20) des flexiblen Schlauchs (2) herum erstreckt, Verpressen des Schlauchabschnitts (20) durch gleichzeitiges Verpressen, durch das Paar von Schlauchhaltern (4a, 4b), eines ersten Verpresselements (M1) der Bandanordnung (B), das dazu geeignet ist, eines der zwei Schlauchteile (TP1) örtlich zu umgeben, und eines zweiten Verpresselements (M2) der Bandanordnung (B), das dazu geeignet ist, das andere der zwei Schlauchteile (TP2) örtlich zu umgeben; und
- Schneiden, durch das Schneidelement (3), des Schlauchabschnitts (20) selektiv an dem Zwischenraum (5) zwischen den Schlauchhaltern (4a, 4b) der Schneidvorrichtung (1), ohne die Verpresselemente (M1, M2) zu schneiden, die jedes der zwei Schnittenden des Schlauchabschnitts in einem geschlossenen Zustand halten.

18. Verwendung nach Anspruch 16 oder 17, wobei die Bandanordnung (B; B') zwei Hälften aufweist, die dazu geeignet sind, aufgrund des Schneidens durch das Schneidelement (3) getrennt zu werden, wobei die zwei Hälften jeweils markiert sind, um jedes der zwei Schlauchteile (TP1, TP2) zusammen mit der entsprechenden Hälfte der Bandanordnung rückverfolgbar zu machen.

## Revendications

1. Dispositif de coupe (1) pour couper un tube flexible (2) au niveau d'une section de tube (20) de celui-ci, le dispositif de coupe (1) comprenant :
- au moins un organe de coupe (3) ;
- un ensemble de bande (B ; B') comprenant un premier organe de sertissage (M1 ; M1') adjacent à un site de coupe de l'ensemble de bande (CS) ;
- un premier support de tube (4a) ;
- un second support de tube (4b), le premier support de tube et le second support de tube étant une paire de supports de tube adaptés pour délimiter un logement (H) destiné à recevoir la section de tube (20) du tube flexible (2) à couper ;
- un interstice (5), situé entre le premier support de tube (4a) et le second support de tube (4b), l'interstice (5) séparant une première région de maintien (C1) associée au premier support de tube (4a) d'une seconde région de maintien (C2) associée au second support de tube (4b) ; et
- un ensemble d'entraînement (6) pour entraîner la paire de supports de tube (4a, 4b) d'une conformation ouverte, permettant l'installation de la section de tube (20) dans le logement (H), à une conformation fermée, dans laquelle le premier support de tube (4a) sertit le premier organe de sertissage (M1) et participe au pincement de la section de tube (20) conjointement avec le second support de tube (4b) ;
dans lequel l'ensemble de bande (B ; B') est adapté pour délimiter, avant tout sertissage, une partie de réception (8) délimitée au moins en partie par une surface (S1) du premier organe de sertissage (M1 ; M1') pour recevoir la section de tube (20) ; et
dans lequel le dispositif de coupe (1) comprend des moyens de positionnement (PM1, PM2) qui sont configurés pour maintenir l'ensemble de bande (B ; B') à l'intérieur du logement (H), dans une configuration interposée pour :
- couvrir la section de tube (20) s'étendant dans la partie de réception (8) d'une part,
- et venir en contact avec au moins l'un parmi le premier support de tube (4a) et le second support de tube (4b) d'autre part, le site de coupe de l'ensemble de bande (CS) étant positionné à une position intermédiaire entre le premier support de tube (4a) et le second support de tube (4b),
moyennant quoi une opération de coupe effectuée pour ladite configuration interposée, par l'organe de coupe (3) à travers l'interstice (5), amène la section de tube (20) à être coupée transversalement au niveau du site de coupe de l'ensemble de bande (CS) sans couper le premier organe de sertissage (M1 ; M1') qui est serti dans la conformation fermée.

2. Dispositif de coupe selon la revendication 1, dans lequel l'ensemble de bande (B) comprend deux organes de sertissage distincts, incluant le premier organe de sertissage (M1) et un second organe de sertissage (M2),
dans lequel, dans la conformation fermée, le premier support de tube (4a) et le second support de tube (4b) sertissent les deux organes de sertissage (M1, M2) et pincent la section de tube (20),
dans lequel la partie de réception (8) pour recevoir la section de tube (20) est délimitée par deux surfaces intérieures (S1, S2), mutuellement espacées et de préférence chacune généralement concave, des deux organes de sertissage (M1, M2), les deux surfaces intérieures incluant la première surface (S1),
et dans lequel les moyens de positionnement (PM1, PM2) sont configurés pour maintenir à l'intérieur du logement (H) l'ensemble de bande (B) en contact avec le premier support de tube (4a) et avec le second support de tube (4b), tandis que l'ensemble de bande (B) s'étend à l'intérieur de la partie de réception (8), les moyens de positionnement (PM1, PM2) coopérant fonctionnellement avec au moins une partie de positionnement de l'ensemble de bande, de préférence avec les deux organes de sertissage (M1, M2), afin d'avoir les deux organes de sertissage (M1, M2) répartis respectivement, de préférence répartis sélectivement, dans la première région de maintien (C1) et dans la seconde région de maintien (C2) ;
moyennant quoi la coupe au niveau du site de coupe de l'ensemble de bande (CS) est une coupe effectuée dans une région intermédiaire entre les deux organes de sertissage (M1, M2) pour couper également la section de tube (20) transversalement sans couper l'un quelconque des deux organes de sertissage (M1, M2) qui sont sertis dans la conformation fermée de la paire de supports de tube (4a, 4b).

3. Dispositif de coupe selon la revendication 2, dans lequel les moyens de positionnement (PM1, PM2) comprennent deux organes magnétiques adaptés pour interagir magnétiquement avec les deux organes de sertissage (M1, M2) constitués chacun d'un matériau magnétisable ou en incluant un,
et dans lequel les organes de sertissage (M1, M2) sont chacun réalisés en une composition malléable et rigide.

4. Dispositif de coupe selon la revendication 2, dans lequel une distance d'espacement minimale entre les deux organes de sertissage (M1, M2), de préférence correspondant à l'interstice (5) juste avant un sertissage dû à un déplacement vers la conformation fermée, est comprise entre 1,5 et 10 mm.
et dans lequel l'ensemble de bande (B) est un dispositif 3-D, chacun des organes de sertissage (M1, M2) étant adapté pour être serti d'une conformation initiale en forme de U à une conformation annulaire qui est aplatie pour maintenir un état scellé au niveau de chaque extrémité coupée correspondante du tube flexible (2).

5. Dispositif de coupe selon la revendication 3 ou 4 lorsqu'elle dépend de la revendication 3, dans lequel les organes de sertissage (M1, M2) définissent chacun une section en U avec un fond (12a), les deux organes magnétiques étant agencés sur un même côté du logement (H) dans la paire de supports de tube (4a, 4b), afin d'attirer chaque fond (12a) des organes de sertissage (M1, M2), le long dudit même côté avant l'installation de la section de tube (20) dans le logement (H).

6. Dispositif de coupe selon la revendication 2, dans lequel les moyens de positionnement (PM1, PM2) comprennent un organe magnétique adapté pour interagir magnétiquement avec au moins l'un parmi les deux organes de sertissage (M1, M2),
et dans lequel les organes de sertissage (M1, M2) sont chacun réalisés en une composition malléable et rigide.

7. Dispositif de coupe selon l'une quelconque des revendications précédentes, dans lequel la paire de supports de tube (4a, 4b) est agencée sensiblement en alignement et espacée le long d'une première direction (D) dans un mode de fonctionnement du dispositif de coupe (1), pour ladite configuration interposée, de sorte que l'opération de coupe effectuée par l'organe de coupe (3) à travers l'interstice (5) et/ou au niveau du site de coupe de l'ensemble de bande (CS) est une coupe de la section de tube (20) perpendiculaire à la première direction (D).

8. Dispositif de coupe selon la revendication 2 seule ou combinée avec l'une quelconque des revendications 3 à 7, dans lequel l'ensemble de bande (B) comprend une bande flexible (11), et dans lequel les deux organes de sertissage (M1, M2) sont reliés par la bande flexible (11), de préférence plus mince que les deux organes de sertissage, laquelle recouvre les deux organes de sertissage (M1, M2) et est pourvue d'une région centrale agencée dans l'interstice (5) et adaptée pour être coupée conjointement avec le tube flexible (2).

9. Dispositif de coupe selon la revendication 2 seule ou combinée avec l'une quelconque des revendications 3 à 7, dans lequel l'ensemble de bande (B) comprend :
- les deux organes de sertissage (M1, M2) qui sont espacés axialement et séparés par un écart ; et
- un ruban pourvu de deux dispositifs d'identification (15, 15'), le ruban interconnectant les deux organes de sertissage (M1, M2) et définissant une portion de jonction (11c) qui est coupée simultanément avec la section de tube (20).

10. Dispositif de coupe selon l'une quelconque des revendications précédentes, comprenant un boîtier (30) qui est fendu ou ouvert sur deux côtés opposés et configuré pour faire passer le tube flexible (2) dans deux passages parallèles, de préférence formés sous forme de fentes (33), tout en étant également mobile le long des deux passages parallèles.

11. Dispositif de coupe selon la revendication 10, dans lequel le boîtier (30) est pourvu d'une partie de guidage pour guider la paire de supports de tube, le premier support de tube (4a) incluant deux premières mâchoires avec une première mâchoire inférieure (J1) et une première mâchoire supérieure (J1'), tandis que le second support de tube (4b) inclut deux secondes mâchoires avec une seconde mâchoire inférieure (J2) et une seconde mâchoire supérieure (J2'),
dans lequel au moins une liaison articulée (HC) relie les deux premières mâchoires ensemble et les deux secondes mâchoires ensemble,
et dans lequel l'au moins une liaison articulée (HC) présente un axe d'articulation (X) et est configurée pour coulisser à l'intérieur du boîtier, ledit axe d'articulation (X) étant mobile linéairement à travers les deux passages parallèles.

12. Dispositif de coupe selon la revendication 10 ou 11, dans lequel l'ensemble d'entraînement (6) comprend un organe d'actionnement (9) mobile par rapport au boîtier (30), parallèlement aux deux passages parallèles,
dans lequel l'organe de coupe (3) est agencé de manière fixe dans le boîtier (30), la section de tube (20) étant coupée dans ladite configuration interposée de l'ensemble de bande (B) en réponse à une action de traction ou de poussée exercée sur l'organe d'actionnement (9) ;

13. Dispositif de coupe selon l'une quelconque des revendications 10 à 12, dans lequel au moins une broche (31, 32) est prévue pour faire coulisser les supports de tube (4a, 4b) par rapport au boîtier (30), ladite broche (31, 32) étant configurée pour permettre un déplacement de la paire de supports de tube (4a, 4b) avant et/ou lors de la coupe de la section de tube (20) par l'organe de coupe (3), la broche (31, 32) étant engagée dans un chemin de guidage (34, 35), le chemin de guidage incluant :
- une première section (30a) de sorte que la broche (31, 32) est déplacée plus près d'un plan (P) qui passe à travers les deux fentes parallèles (33), moyennant quoi le logement (H) diminue de taille pour permettre un sertissage de la section de tube (20) reçue dans le logement (H) par les mâchoires (J1, J1', J2, J2') ;
- une seconde section (30b) qui est parallèle aux deux fentes parallèles (33), permettant à la section de tube (20) déjà sertie d'être déplacée par rapport à l'organe de coupe (3).

14. Dispositif de coupe selon l'une quelconque des revendications précédentes, dans lequel l'ensemble de bande (B) présente une longueur (L), qui est une longueur d'un ruban ou d'une bande flexible et allongée (11), qui est supérieure à une longueur (L1) du logement (H).

15. Dispositif de coupe selon l'une quelconque des revendications précédentes, dans lequel chacun des organes de sertissage (M1, M2) est pourvu de saillies ou de reliefs qui coopèrent mécaniquement, de préférence sans aucune interaction adhésive, avec les supports de tube (4a, 4b).

16. Utilisation du dispositif de coupe (1) selon la revendication 1, dans laquelle l'ensemble de bande (B ; B') est couplé à une section de tube autour d'une surface extérieure de celle-ci pour permettre de couper un tube flexible (2) au niveau de la section de tube par le dispositif de coupe (1), de sorte qu'une extrémité coupée est obtenue respectivement au niveau de deux portions de tube (TP1, TP2) du tube flexible (2), qui sert à la circulation d'un fluide biopharmaceutique, dans laquelle le dispositif de coupe (1) est utilisé pour fournir une déconnexion lors de la séparation des deux portions de tube (TP1, TP2) par :
- le sertissage d'une section de tube (20) du tube flexible en sertissant, par le premier support de tube (4a), un premier organe de sertissage (M1 ; M1') de l'ensemble de bande (B ; B1') apte à entourer localement une première des portions de tube (TP1), tandis que l'autre des portions de tube (TP2) est scellée via une action de sertissage impliquant le second support de tube (4b) ; et
- la coupe, par l'organe de coupe (3), de la section de tube (20) sélectivement au niveau d'un site de coupe de l'ensemble de bande (CS) de l'ensemble de bande, qui est adjacent au premier organe de sertissage (M1 ; M1') serti par le premier support de tube (4a) d'une part et adjacent au second support de tube d'autre part, sans couper le premier organe de sertissage (M1 ; M1') qui maintient l'extrémité coupée de ladite première portion de tube (TP1) dans un état fermé.

17. Utilisation du dispositif de coupe selon la revendication 2, dans laquelle l'ensemble de bande (B) permet de couper un tube flexible (2) par le dispositif de coupe (1), de sorte qu'une extrémité coupée est obtenue respectivement au niveau de deux portions de tube (TP1, TP2) du tube flexible (2), qui sert à la circulation d'un fluide biopharmaceutique, dans laquelle le dispositif de coupe (1) est utilisé pour fournir une déconnexion stérile lors de la séparation des deux portions de tube (TP1, TP2) par :
- dans un état couplé dans lequel l'ensemble de bande (B) s'étend autour d'une section de tube (20) du tube flexible (2), le sertissage de ladite section de tube (20) en sertissant simultanément, par la paire de supports de tube (4a, 4b), un premier organe de sertissage (M1) de l'ensemble de bande (B) apte à entourer localement l'une des deux portions de tube (TP1) et un second organe de sertissage (M2) de l'ensemble de bande (B) apte à entourer localement l'autre des deux portions de tube (TP2) ; et
- la coupe, par l'organe de coupe (3), de la section de tube (20) sélectivement au niveau de l'interstice (5) entre les supports de tube (4a, 4b) du dispositif de coupe (1) sans couper les organes de sertissage (M1, M2) qui maintiennent chacune des deux extrémités coupées de la section de tube dans un état fermé.

18. Utilisation selon la revendication 16 ou 17, dans laquelle l'ensemble de bande (B ; B') présente deux moitiés adaptées pour être séparées du fait de la coupe par l'organe de coupe (3), les deux moitiés étant chacune marquée pour rendre chacune des deux portions de tube (TP1, TP2) traçable conjointement avec la moitié d'ensemble de bande correspondante.
